# EUROPÄISCHE PATENTSCHRIFT

(11) **EP 2 438 182 B1**
(45) Veröffentlichungstag und Bekanntmachung des Hinweises auf die Patenterteilung: **10.10.2018**
(21) Anmeldenummer: 10724460.0
(22) Anmeldetag: 02.06.2010
(51) Int. Cl.: C12P 17/04, C12N 9/90

(54) **BIOKATALYTISCHE HERSTELLUNG VON AMBROXAN**
BIOCATALYTIC PRODUCTION OF AMBROXAN
PRODUCTION BIOCATALYTIQUE D'AMBROXANE

(30) Priorität: 05.06.2009 EP 09162104
(43) Veröffentlichungstag der Anmeldung: 11.04.2012
(60) Teilanmeldung: 18179368.8
(73) Patentinhaber: BASF SE, 67056 Ludwigshafen am Rhein (DE)
(72) Erfinder: BREUER, Michael, 64285 Darmstadt (DE); HÖRSTER, Andrea, 67067 Ludwigshafen (DE); HAUER, Bernhard, 67136 Fußgönheim (DE)
(74) Vertreter: BASF IP Association
(86) Internationale Anmeldenummer: PCT/EP2010/057696
(87) Internationale Veröffentlichungsnummer: WO 2010/139719

(56) Entgegenhaltungen:
- JP-A- 2009 060 799
- NEUMANN S ET AL: "Purification, partial characterization and substrate specificity of a squalene Cyclase from Bacillus acidocaldarius", BIOLOGICAL CHEMISTRY HOPPE-SEYLER, WALTER DE GRUYTER, BERLIN, DE, Bd. 367, 1. August 1986 (1986-08-01), Seiten 723-729, XP008128101, ISSN: 0177-3593 in der Anmeldung erwähnt
- REIPEN I G ET AL: "Zymomonas mobilis squalene-hopene cyclase gene (shc): cloning, DNA sequence analysis and expression in escherichia coli", MICROBIOLOGY, SOCIETY FOR GENERAL MICROBIOLOGY, READING, GB, Bd. 141, Nr. 1, 1. Januar 1995 (1995-01-01), Seiten 155-161, XP002969317, ISSN: 1350-0872
- PERZL M ET AL: "Squalene-hopene cyclase from Bradyrhizobium japonicum: Cloning, expression, sequence analysis and comparison to triterpenoid cyclases", MICROBIOLOGY, SOCIETY FOR GENERAL MICROBIOLOGY, READING, GB, Bd. 143, Nr. 4, 1. Januar 1997 (1997-01-01), Seiten 1235-1242, XP000993224, ISSN: 1350-0872
- TIPPELT A ET AL: "Squalene-hopene cyclase from Methylococcus capsulatus (Bath): a bacterium producing hopanoids and steroids", BIOCHIMICA ET BIOPHYSICA ACTA - LIPIDS AND LIPID METABOLISM, ELSEVIER SCIENCE BV. AMSTERDAM, NL, Bd. 1391, Nr. 2, 1. Januar 1998 (1998-01-01), Seiten 223-232, XP002969318, ISSN: 0005-2760, DOI: 10.1016/S0005-2760(97)00212-9
- OCHS D ET AL: "CLONING, EXPRESSION, AND SEQUENCING OF SQUALENCE-HOPENE CYCLASE, A KEY ENZYME IN TRITERPENOID METABOLISM", JOURNAL OF BACTERIOLOGY, AMERICAN SOCIETY FOR MICROBIOLOGY, WASHINGTON, DC; US, Bd. 174, Nr. 1, 1. Januar 1992 (1992-01-01), Seiten 298-302, XP000992925, ISSN: 0021-9193
- SATO T ET AL: "Overexpression of squalene-hopene cyclase by the pET vector in Escherichia coli and first identification of tryptophan and aspartic acid residues inside the QW motif as active sites.", BIOSCIENCE, BIOTECHNOLOGY, AND BIOCHEMISTRY, Bd. 62, Nr. 2, Februar 1998 (1998-02), Seiten 407-411, XP002607431, ISSN: 0916-8451
- SNOWDEN R L ET AL: "INTERNAL NUCLEOPHILIC TERMINATION IN BIOMIMETIC ACID MEDIATED POLYENE CYCLIZATIONS: STEREOCHEMICAL AND MECHANISTIC IMPLICATIONS. SYNTHESIS OF ( )-AMBROX AND ITS DIASTEREOISOMERS", JOURNAL OF ORGANIC CHEMISTRY, AMERICAN CHEMICAL SOCIETY, EASTON.; US, Bd. 57, Nr. 3, 31. Januar 1992 (1992-01-31), Seiten 955-960, XP000561987, ISSN: 0022-3263, DOI: 10.1021/JO00029A031
- OGURA K ET AL: "A NOVEL SUBSTRATE FOR PRENYL TRANSFERASE. FORMATION OF A NONALLYLIC CIS HOMO FARNESYL PYRO PHOSPHATE", JOURNAL OF THE AMERICAN CHEMICAL SOCIETY, Bd. 96, Nr. 12, 1974, Seiten 4037-4038, XP002607432, ISSN: 0002-7863
- DATABASE UniProt [Online] 1. Februar 2005 (2005-02-01), "Squalene-hopene cyclase (Zymomonas mobilis)", XP002607434, Database accession no. Q5NM88
- DATABASE UniProt [Online] 12. Juni 2007 (2007-06-12), "Squalene-hopene cyclase (Bradyrhizobium sp.)", XP002607435, Database accession no. A5EBP6
- DATABASE UniProt [Online] 17. Oktober 2006 (2006-10-17), "Squalene-hopene cyclase (Burkholderia ambifaria)", XP002607436, Database accession no. Q0B5S3
- DATABASE UniProt [Online] 17. Oktober 2006 (2006-10-17), "Squalene-hopene synthase (Burkholderia ambifaria)", XP002607437, Database accession no. Q0B2H5
- DATABASE UniProt [Online] 2. September 2008 (2008-09-02), "Putative squalene-hopene cyclase (Bacillus anthracis)", XP002607438, Database accession no. B3IZ59
- DATABASE UniProt [Online] 14. November 2006 (2006-11-14), "Putative two-component system sensor kinase (Frankia alni)", XP002607439, Database accession no. Q05HK2
- DATABASE UniProt [Online] 13. Oktober 2006 (2006-10-13), "Squalene-hopene cyclase (Rhodopseudomonas palustris)", XP002607440, Database accession no. Q07I43
- DATABASE UniProt [Online] 1. November 1999 (1999-11-01), "Putative squalene-hopene cyclase (Streptomyces coelicolor)", XP002607441, Database accession no. Q9X7V9

### SEQUENCE LISTING

<110> BASF SE
<120> Biokatalytische Herstellung von Ambroxan
<130> PF 62207 PCT
<160> 11
<170> PatentIn version 3.5
<210> 1
   <211> 6525
   <212> DNA
   <213> Zymomonas mobilis
<220>
   <221> CDS
   <222> (154)..(2328)
<400> 1
   cgatcaccac aattcagcaa attgtgaaca tcatcacgtt catctttccc tggttgccaa       60
   tggcccattt tcctgtcagt aacgagaagg tcgcgaattc aggcgctttt tagactggtc      120
   gtaatgaaca attcttaaga aggagatata cat atg ggt att gac aga atg aat       174
                                        Met Gly Ile Asp Arg Met Asn
                                        1               5
   agc tta agt cgc ttg tta atg aag aag att ttc ggg gct gaa aaa acc        222
   Ser Leu Ser Arg Leu Leu Met Lys Lys Ile Phe Gly Ala Glu Lys Thr
           10                  15                  20
   tcg tat aaa ccg gct tcc gat acc ata atc gga acg gat acc ctg aaa        270
   Ser Tyr Lys Pro Ala Ser Asp Thr Ile Ile Gly Thr Asp Thr Leu Lys
       25                  30                  35
   aga ccg aac cgg cgg cct gaa ccg acg gca aaa gtc gac aaa acg ata        318
   Arg Pro Asn Arg Arg Pro Glu Pro Thr Ala Lys Val Asp Lys Thr Ile
   40                  45                  50                  55
   ttc aag act atg ggg aat agt ctg aat aat acc ctt gtt tca gcc tgt        366
   Phe Lys Thr Met Gly Asn Ser Leu Asn Asn Thr Leu Val Ser Ala Cys
                   60                  65                  70
   gac tgg ttg atc gga caa caa aag ccc gat ggt cat tgg gtc ggt gcc        414
   Asp Trp Leu Ile Gly Gln Gln Lys Pro Asp Gly His Trp Val Gly Ala
               75                  80                  85
   gtg gaa tcc aat gct tcg atg gaa gca gaa tgg tgt ctg gcc ttg tgg        462
   Val Glu Ser Asn Ala Ser Met Glu Ala Glu Trp Cys Leu Ala Leu Trp
           90                  95                  100
   ttt ttg ggt ctg gaa gat cat ccg ctt cgt cca aga ttg ggc aat gct        510
   Phe Leu Gly Leu Glu Asp His Pro Leu Arg Pro Arg Leu Gly Asn Ala
       105                 110                 115
   ctt ttg gaa atg cag cgg gaa gat ggc tct tgg gga gtc tat ttc ggc        558
   Leu Leu Glu Met Gln Arg Glu Asp Gly Ser Trp Gly Val Tyr Phe Gly
   120                 125                 130                 135
   gct gga aat ggc gat atc aat gcc acg gtt gaa gcc tat gcg gcc ttg        606
   Ala Gly Asn Gly Asp Ile Asn Ala Thr Val Glu Ala Tyr Ala Ala Leu
                   140                 145                 150
   cgg tct ttg ggg tat tct gcc gat aat cct gtt ttg aaa aaa gcg gca        654
   Arg Ser Leu Gly Tyr Ser Ala Asp Asn Pro Val Leu Lys Lys Ala Ala
               155                 160                 165
   gca tgg att gct gaa aaa ggc gga tta aaa aat atc cgt gtc ttt acc        702
   Ala Trp Ile Ala Glu Lys Gly Gly Leu Lys Asn Ile Arg Val Phe Thr
           170                 175                 180
   cgt tat tgg ctg gcg ttg atc ggg gaa tgg cct tgg gaa aag acc cct        750
   Arg Tyr Trp Leu Ala Leu Ile Gly Glu Trp Pro Trp Glu Lys Thr Pro
       185                 190                 195
   aac ctt ccc cct gaa att atc tgg ttc cct gat aat ttt gtc ttt tcg        798
   Asn Leu Pro Pro Glu Ile Ile Trp Phe Pro Asp Asn Phe Val Phe Ser
   200                 205                 210                 215
   att tat aat ttt gcc caa tgg gcg cgg gca acc atg gtg ccg att gct        846
   Ile Tyr Asn Phe Ala Gln Trp Ala Arg Ala Thr Met Val Pro Ile Ala
                   220                 225                 230
   att ctg tcc gcg aga cga cca agc cgc ccg ctg cgc cct caa gac cga        894
   Ile Leu Ser Ala Arg Arg Pro Ser Arg Pro Leu Arg Pro Gln Asp Arg
               235                 240                 245
   ttg gat gaa ctg ttt cca gaa ggc cgc gct cgc ttt gat tat gaa ttg        942
   Leu Asp Glu Leu Phe Pro Glu Gly Arg Ala Arg Phe Asp Tyr Glu Leu
           250                 255                 260
   ccg aaa aaa gaa ggc atc gat ctt tgg tcg caa ttt ttc cga acc act        990
   Pro Lys Lys Glu Gly Ile Asp Leu Trp Ser Gln Phe Phe Arg Thr Thr
       265                 270                 275
   gac cgt gga tta cat tgg gtt cag tcc aat ctg tta aag cgc aat agc       1038
   Asp Arg Gly Leu His Trp Val Gln Ser Asn Leu Leu Lys Arg Asn Ser
   280                 285                 290                 295
   ttg cgt gaa gcc gct atc cgt cat gtt ttg gaa tgg att atc cgg cat       1086
   Leu Arg Glu Ala Ala Ile Arg His Val Leu Glu Trp Ile Ile Arg His
                   300                 305                 310
   cag gat gcc gat ggc ggt tgg ggt gga att cag cca cct tgg gtc tat       1134
   Gln Asp Ala Asp Gly Gly Trp Gly Gly Ile Gln Pro Pro Trp Val Tyr
               315                 320                 325
   ggt ttg atg gcg tta cat ggt gaa ggc tat cag ctt tat cat ccg gtg       1182
   Gly Leu Met Ala Leu His Gly Glu Gly Tyr Gln Leu Tyr His Pro Val
           330                 335                 340
   atg gcc aag gct ttg tcg gct ttg gat gat ccc ggt tgg cga cat gac       1230
   Met Ala Lys Ala Leu Ser Ala Leu Asp Asp Pro Gly Trp Arg His Asp
       345                 350                 355
   aga ggc gag tct tct tgg ata cag gcc acc aat agt ccg gta tgg gat       1278
   Arg Gly Glu Ser Ser Trp Ile Gln Ala Thr Asn Ser Pro Val Trp Asp
   360                 365                 370                 375
   aca atg ttg gcc ttg atg gcg tta aaa gac gcc aag gcc gag gat cgt       1326
   Thr Met Leu Ala Leu Met Ala Leu Lys Asp Ala Lys Ala Glu Asp Arg
                   380                 385                 390
   ttt acg ccg gaa atg gat aag gcc gcc gat tgg ctt ttg gct cga cag        1374
   Phe Thr Pro Glu Met Asp Lys Ala Ala Asp Trp Leu Leu Ala Arg Gln
               395                 400                 405
   gtc aaa gtc aaa ggc gat tgg tca atc aaa ctg ccc gat gtt gaa ccc        1422
   Val Lys Val Lys Gly Asp Trp Ser Ile Lys Leu Pro Asp Val Glu Pro
           410                 415                 420
   ggt gga tgg gca ttt gaa tat gcc aat gat cgc tat ccc gat acc gat        1470
   Gly Gly Trp Ala Phe Glu Tyr Ala Asn Asp Arg Tyr Pro Asp Thr Asp
       425                 430                 435
   gat acc gcc gtc gct ttg atc gcc ctt tcc tct tat cgt gat aag gag        1518
   Asp Thr Ala Val Ala Leu Ile Ala Leu Ser Ser Tyr Arg Asp Lys Glu
   440                 445                 450                 455
   gag tgg caa aag aaa ggc gtt gag gac gcc att acc cgt ggg gtt aat        1566
   Glu Trp Gln Lys Lys Gly Val Glu Asp Ala Ile Thr Arg Gly Val Asn
                   460                 465                 470
   tgg ttg atc gcc atg caa agc gaa tgt ggc ggt tgg gga gcc ttt gat        1614
   Trp Leu Ile Ala Met Gln Ser Glu Cys Gly Gly Trp Gly Ala Phe Asp
               475                 480                 485
   aag gat aat aac aga agt atc ctt tcc aaa att cct ttt tgt gat ttc        1662
   Lys Asp Asn Asn Arg Ser Ile Leu Ser Lys Ile Pro Phe Cys Asp Phe
           490                 495                 500
   gga gaa tct att gat ccg cct tca gtc gat gta acg gcg cat gtt tta        1710
   Gly Glu Ser Ile Asp Pro Pro Ser Val Asp Val Thr Ala His Val Leu
       505                 510                 515
   gag gcc ttt ggc acc ttg gga ctg tcc cgc gat atg ccg gtc atc caa        1758
   Glu Ala Phe Gly Thr Leu Gly Leu Ser Arg Asp Met Pro Val Ile Gln
   520                 525                 530                 535
   aaa gcg atc gac tat gtc cgt tcc gaa cag gaa gcc gaa ggc gcg tgg        1806
   Lys Ala Ile Asp Tyr Val Arg Ser Glu Gln Glu Ala Glu Gly Ala Trp
                   540                 545                 550
   ttt ggt cgt tgg ggc gtt aat tat atc tat ggc acc ggt gcg gtt ctg        1854
   Phe Gly Arg Trp Gly Val Asn Tyr Ile Tyr Gly Thr Gly Ala Val Leu
               555                 560                 565
   cct gct ttg gcg gcg atc ggt gaa gat atg acc cag cct tac atc acc        1902
   Pro Ala Leu Ala Ala Ile Gly Glu Asp Met Thr Gln Pro Tyr Ile Thr
           570                 575                 580
   aag gct tgc gat tgg ctg gtc gca cat cag cag gaa gac ggc ggt tgg        1950
   Lys Ala Cys Asp Trp Leu Val Ala His Gln Gln Glu Asp Gly Gly Trp
       585                 590                 595
   ggc gaa agc tgc tct tcc tat atg gag att gat tcc att ggg aag ggc        1998
   Gly Glu Ser Cys Ser Ser Tyr Met Glu Ile Asp Ser Ile Gly Lys Gly
   600                 605                 610                 615
   cca acc acg ccg tcc cag act gct tgg gct ttg atg ggg ttg atc gcg        2046
   Pro Thr Thr Pro Ser Gln Thr Ala Trp Ala Leu Met Gly Leu Ile Ala
                   620                 625                 630
   gcc aat cgt ccc gaa gat tat gaa gcc att gcc aag gga tgc cat tat        2094
   Ala Asn Arg Pro Glu Asp Tyr Glu Ala Ile Ala Lys Gly Cys His Tyr
               635                 640                 645
   ctg att gat cgc caa gag cag gat ggt agc tgg aaa gaa gaa gaa ttc      2142
   Leu Ile Asp Arg Gln Glu Gln Asp Gly Ser Trp Lys Glu Glu Glu Phe
           650                 655                 660
   acc ggc acc gga ttc ccc ggt tat ggc gtg ggt cag acg atc aag ttg      2190
   Thr Gly Thr Gly Phe Pro Gly Tyr Gly Val Gly Gln Thr Ile Lys Leu
       665                 670                 675
   gat gat ccg gct tta tcg aaa cga ttg ctt caa ggc gct gaa ctg tca      2238
   Asp Asp Pro Ala Leu Ser Lys Arg Leu Leu Gln Gly Ala Glu Leu Ser
   680                 685                 690                 695
   cgg gcg ttt atg ctg cgt tat gat ttt tat cgg caa ttc ttc ccg att      2286
   Arg Ala Phe Met Leu Arg Tyr Asp Phe Tyr Arg Gln Phe Phe Pro Ile
                   700                 705                 710
   atg gcg tta agt cgg gca gag aga ctg att gat ttg aat aat              2328
   Met Ala Leu Ser Arg Ala Glu Arg Leu Ile Asp Leu Asn Asn
               715                 720                 725
   tgatagtatt ggggcggagg agtcttttta aaagagacta ctccgtccta ttttcgagga     2388
   tccgtcgacc tgcagccaag cttggctgtt ttggcggatg agagaagatt ttcagcctga     2448
   tacagattaa atcagaacgc agaagcggtc tgataaaaca gaatttgcct ggcggcagta     2508
   gcgcggtggt cccacctgac cccatgccga actcagaagt gaaacgccgt agcgccgatg     2568
   gtagtgtggg gtctccccat gcgagagtag ggaactgcca ggcatcaaat aaaacgaaag     2628
   gctcagtcga aagactgggc ctttcgtttt atctgttgtt tgtcggtgaa cgctctcctg     2688
   agtaggacaa atccgccggg agcggatttg aacgttgcga agcaacggcc cggagggtgg     2748
   cgggcaggac gcccgccata aactgccagg catcaaatta agcagaaggc catcctgacg     2808
   gatggccttt ttgcgtttct acaaactctt ttgtttattt ttctaaatac attcaaatat     2868
   gtatccgctc atgagacaat aaccctgata aatgcttcaa taatattgaa aaaggaagag     2928
   tatgagtatt caacatttcc gtgtcgccct tattcccttt tttgcggcat tttgccttcc     2988
   tgtttttgct cacccagaaa cgctggtgaa agtaaaagat gctgaagatc agttgggtgc     3048
   acgagtgggt tacatcgaac tggatctcaa cagcggtaag atccttgaga gttttcgccc     3108
   cgaagaacgt tttccaatga tgagcacttt taaagttctg ctatgtggcg cggtattatc     3168
   ccgtgttgac gccgggcaag agcaactcgg tcgccgcata cactattctc agaatgactt     3228
   ggttgagtac tcaccagtca cagaaaagca tcttacggat ggcatgacag taagagaatt     3288
   atgcagtgct gccataacca tgagtgataa cactgcggcc aacttacttc tgacaacgat     3348
   cggaggaccg aaggagctaa ccgctttttt gcacaacatg ggggatcatg taactcgcct     3408
   tgatcgttgg gaaccggagc tgaatgaagc cataccaaac gacgagcgtg acaccacgat     3468
   gcctgtagca atggcaacaa cgttgcgcaa actattaact ggcgaactac ttactctagc     3528
   ttcccggcaa caattaatag actggatgga ggcggataaa gttgcaggac cacttctgcg     3588
   ctcggccctt ccggctggct ggtttattgc tgataaatct ggagccggtg agcgtgggtc 3648
   tcgcggtatc attgcagcac tggggccaga tggtaagccc tcccgtatcg tagttatcta 3708
   cacgacgggg agtcaggcaa ctatggatga acgaaataga cagatcgctg agataggtgc 3768
   ctcactgatt aagcattggt aactgtcaga ccaagtttac tcatatatac tttagattga 3828
   tttaaaactt catttttaat ttaaaaggat ctaggtgaag atcctttttg ataatctcat 3888
   gaccaaaatc ccttaacgtg agttttcgtt ccactgagcg tcagaccccg tagaaaagat 3948
   caaaggatct tcttgagatc ctttttttct gcgcgtaatc tgctgcttgc aaacaaaaaa 4008
   accaccgcta ccagcggtgg tttgtttgcc ggatcaagag ctaccaactc tttttccgaa 4068
   ggtaactggc ttcagcagag cgcagatacc aaatactgtc cttctagtgt agccgtagtt 4128
   aggccaccac ttcaagaact ctgtagcacc gcctacatac ctcgctctgc taatcctgtt 4188
   accagtggct gctgccagtg gcgataagtc gtgtcttacc gggttggact caagacgata 4248
   gttaccggat aaggcgcagc ggtcgggctg aacggggggt tcgtgcacac agcccagctt 4308
   ggagcgaacg acctacaccg aactgagata cctacagcgt gagctatgag aaagcgccac 4368
   gcttcccgaa gggagaaagg cggacaggta tccggtaagc ggcagggtcg gaacaggaga 4428
   gcgcacgagg gagcttccag ggggaaacgc ctggtatctt tatagtcctg tcgggtttcg 4488
   ccacctctga cttgagcgtc gatttttgtg atgctcgtca ggggggcgga gcctatggaa 4548
   aaacgccagc aacgcggcct ttttacggtt cctggccttt tgctggcctt ttgctcacat 4608
   gttctttcct gcgttatccc ctgattctgt ggataaccgt attaccgcct ttgagtgagc 4668
   tgataccgct cgccgcagcc gaacgaccga gcgcagcgag tcagtgagcg aggaagcgga 4728
   agagcgcctg atgcggtatt ttctccttac gcatctgtgc ggtatttcac accgcatata 4788
   tggtgcactc tcagtacaat ctgctctgat gccgcatagt taagccagta tacactccgc 4848
   tatcgctacg tgactgggtc atggctgcgc cccgacaccc gccaacaccc gctgacgcgc 4908
   cctgacgggc ttgtctgctc ccggcatccg cttacagaca agctgtgacc gtctccggga 4968
   gctgcatgtg tcagaggttt tcaccgtcat caccgaaacg cgcgaggcag ctgcggtaaa 5028
   gctcatcagc gtggtcgtga agcgattcac agatgtctgc ctgttcatcc gcgtccagct 5088
   cgttgagttt ctccagaagc gttaatgtct ggcttctgat aaagcgggcc atgttaaggg 5148
   cggttttttc ctgtttggtc actgatgcct ccgtgtaagg gggatttctg ttcatggggg 5208
   taatgatacc gatgaaacga gagaggatgc tcacgatacg ggttactgat gatgaacatg 5268
   cccggttact ggaacgttgt gagggtaaac aactggcggt atggatgcgg cgggaccaga 5328
   gaaaaatcac tcagggtcaa tgccagcgct tcgttaatac agatgtaggt gttccacagg 5388
   gtagccagca gcatcctgcg atgcagatcc ggaacataat ggtgcagggc gctgacttcc 5448
   gcgtttccag actttacgaa acacggaaac cgaagaccat tcatgttgtt gctcaggtcg 5508
   cagacgtttt gcagcagcag tcgcttcacg ttcgctcgcg tatcggtgat tcattctgct 5568
   aaccagtaag gcaaccccgc cagcctagcc gggtcctcaa cgacaggagc acgatcatgc 5628
   gcacccgtgg ccaggaccca acgctgcccg agatgcgccg cgtgcggctg ctggagatgg 5688
   cggacgcgat ggatatgttc tgccaagggt tggtttgcgc attcacagtt ctccgcaaga 5748
   attgattggc tccaattctt ggagtggtga atccgttagc gaggtgccgc cggcttccat 5808
   tcaggtcgag gtggcccggc tccatgcacc gcgacgcaac gcggggaggc agacaaggta 5868
   tagggcggcg cctacaatcc atgccaaccc gttccatgtg ctcgccgagg cggcataaat 5928
   cgccgtgacg atcagcggtc caatgatcga agttaggctg gtaagagccg cgagcgatcc 5988
   ttgaagctgt ccctgatggt cgtcatctac ctgcctggac agcatggcct gcaacgcggg 6048
   catcccgatg ccgccggaag cgagaagaat cataatgggg aaggccatcc agcctcgcgt 6108
   cgcgaacgcc agcaagacgt agcccagcgc gtcggccgcc atgccggcga taatggcctg 6168
   cttctcgccg aaacgtttgg tggcgggacc agtgacgaag gcttgagcga gggcgtgcaa 6228
   gattccgaat accgcaagcg acaggccgat catcgtcgcg ctccagcgaa agcggtcctc 6288
   gccgaaaatg acccagagcg ctgccggcac ctgtcctacg agttgcatga taaagaagac 6348
   agtcataagt gcggcgacga tagtcatgcc ccgcgcccac cggaaggagc tgactgggtt 6408
   gaaggctctc aagggcatcg gtcgacgctc tcccttatgc gactcctgca ttaggaagca 6468
   gcccagtagt aggttgaggc cgttgagcac cgccgccgca aggaatggtg catgcat 6525
<210> 2
   <211> 725
   <212> PRT
   <213> Zymomonas mobilis
<400> 2
   Met Gly Ile Asp Arg Met Asn Ser Leu Ser Arg Leu Leu Met Lys Lys
   1 5 10 15
   Ile Phe Gly Ala Glu Lys Thr Ser Tyr Lys Pro Ala Ser Asp Thr Ile
   20 25 30
   Ile Gly Thr Asp Thr Leu Lys Arg Pro Asn Arg Arg Pro Glu Pro Thr
   35 40 45
   Ala Lys Val Asp Lys Thr Ile Phe Lys Thr Met Gly Asn Ser Leu Asn
   50 55 60
   Asn Thr Leu Val Ser Ala Cys Asp Trp Leu Ile Gly Gln Gln Lys Pro
   65 70 75 80
   Asp Gly His Trp Val Gly Ala Val Glu Ser Asn Ala Ser Met Glu Ala
                   85                  90                  95
   Glu Trp Cys Leu Ala Leu Trp Phe Leu Gly Leu Glu Asp His Pro Leu
               100                 105                 110
   Arg Pro Arg Leu Gly Asn Ala Leu Leu Glu Met Gln Arg Glu Asp Gly
           115                 120                 125
   Ser Trp Gly Val Tyr Phe Gly Ala Gly Asn Gly Asp Ile Asn Ala Thr
       130                 135                 140
   Val Glu Ala Tyr Ala Ala Leu Arg Ser Leu Gly Tyr Ser Ala Asp Asn
   145                 150                 155                 160
   Pro Val Leu Lys Lys Ala Ala Ala Trp Ile Ala Glu Lys Gly Gly Leu
                   165                 170                 175
   Lys Asn Ile Arg Val Phe Thr Arg Tyr Trp Leu Ala Leu Ile Gly Glu
               180                 185                 190
   Trp Pro Trp Glu Lys Thr Pro Asn Leu Pro Pro Glu Ile Ile Trp Phe
           195                 200                 205
   Pro Asp Asn Phe Val Phe Ser Ile Tyr Asn Phe Ala Gln Trp Ala Arg
       210                 215                 220
   Ala Thr Met Val Pro Ile Ala Ile Leu Ser Ala Arg Arg Pro Ser Arg
   225                 230                 235                 240
   Pro Leu Arg Pro Gln Asp Arg Leu Asp Glu Leu Phe Pro Glu Gly Arg
                   245                 250                 255
   Ala Arg Phe Asp Tyr Glu Leu Pro Lys Lys Glu Gly Ile Asp Leu Trp
               260                 265                 270
   Ser Gln Phe Phe Arg Thr Thr Asp Arg Gly Leu His Trp Val Gln Ser
           275                 280                 285
   Asn Leu Leu Lys Arg Asn Ser Leu Arg Glu Ala Ala Ile Arg His Val
       290                 295                 300
   Leu Glu Trp Ile Ile Arg His Gln Asp Ala Asp Gly Gly Trp Gly Gly
   305                 310                 315                 320
   Ile Gln Pro Pro Trp Val Tyr Gly Leu Met Ala Leu His Gly Glu Gly
                   325                 330                 335
   Tyr Gln Leu Tyr His Pro Val Met Ala Lys Ala Leu Ser Ala Leu Asp
               340                 345                 350
   Asp Pro Gly Trp Arg His Asp Arg Gly Glu Ser Ser Trp Ile Gln Ala
           355                 360                 365
   Thr Asn Ser Pro Val Trp Asp Thr Met Leu Ala Leu Met Ala Leu Lys
       370                 375                 380
   Asp Ala Lys Ala Glu Asp Arg Phe Thr Pro Glu Met Asp Lys Ala Ala
   385                 390                 395                 400
   Asp Trp Leu Leu Ala Arg Gln Val Lys Val Lys Gly Asp Trp Ser Ile
                   405                 410                 415
   Lys Leu Pro Asp Val Glu Pro Gly Gly Trp Ala Phe Glu Tyr Ala Asn
               420                 425                 430
   Asp Arg Tyr Pro Asp Thr Asp Asp Thr Ala Val Ala Leu Ile Ala Leu
           435                 440                 445
   Ser Ser Tyr Arg Asp Lys Glu Glu Trp Gln Lys Lys Gly Val Glu Asp
       450                 455                 460
   Ala Ile Thr Arg Gly Val Asn Trp Leu Ile Ala Met Gln Ser Glu Cys
   465                 470                 475                 480
   Gly Gly Trp Gly Ala Phe Asp Lys Asp Asn Asn Arg Ser Ile Leu Ser
                   485                 490                 495
   Lys Ile Pro Phe Cys Asp Phe Gly Glu Ser Ile Asp Pro Pro Ser Val
               500                 505                 510
   Asp Val Thr Ala His Val Leu Glu Ala Phe Gly Thr Leu Gly Leu Ser
           515                 520                 525
   Arg Asp Met Pro Val Ile Gln Lys Ala Ile Asp Tyr Val Arg Ser Glu
       530                 535                 540
   Gln Glu Ala Glu Gly Ala Trp Phe Gly Arg Trp Gly Val Asn Tyr Ile
   545                 550                 555                 560
   Tyr Gly Thr Gly Ala Val Leu Pro Ala Leu Ala Ala Ile Gly Glu Asp
                   565                 570                 575
   Met Thr Gln Pro Tyr Ile Thr Lys Ala Cys Asp Trp Leu Val Ala His
                   580                 585                 590
   Gln Gln Glu Asp Gly Gly Trp Gly Glu Ser Cys Ser Ser Tyr Met Glu
           595                 600                 605
   Ile Asp Ser Ile Gly Lys Gly Pro Thr Thr Pro Ser Gln Thr Ala Trp
       610                 615                 620
   Ala Leu Met Gly Leu Ile Ala Ala Asn Arg Pro Glu Asp Tyr Glu Ala
   625                 630                 635                 640
   Ile Ala Lys Gly Cys His Tyr Leu Ile Asp Arg Gln Glu Gln Asp Gly
                   645                 650                 655
   Ser Trp Lys Glu Glu Glu Phe Thr Gly Thr Gly Phe Pro Gly Tyr Gly
               660                 665                 670
   Val Gly Gln Thr Ile Lys Leu Asp Asp Pro Ala Leu Ser Lys Arg Leu
           675                 680                 685
   Leu Gln Gly Ala Glu Leu Ser Arg Ala Phe Met Leu Arg Tyr Asp Phe
       690                 695                 700
   Tyr Arg Gln Phe Phe Pro Ile Met Ala Leu Ser Arg Ala Glu Arg Leu
   705                 710                 715                 720
   Ile Asp Leu Asn Asn
                   725
<210> 3
   <211> 25
   <212> DNA
   <213> Artificial Sequence
<220>
   <223> Primer
<400> 3
   gcgctgtttc atatgggtat tgaca 25
<210> 4
   <211> 25
   <212> DNA
   <213> Artificial Sequence
<220>
   <223> Primer
<400> 4
   gcgcttaccc tggatcctcg aaaat 25
<210> 5
   <211> 684
   <212> PRT
   <213> Bradyrhizobium japonicum
<400> 5
   Met Thr Val Thr Ser Ser Ala Ser Ala Arg Ala Thr Arg Asp Pro Gly
   1               5                   10                  15
   Asn Tyr Gln Thr Ala Leu Gln Ser Thr Val Arg Ala Ala Ala Asp Trp
               20                  25                  30
   Leu Ile Ala Asn Gln Lys Pro Asp Gly His Trp Val Gly Arg Ala Glu
           35                  40                  45
   Ser Asn Ala Cys Met Glu Ala Gln Trp Cys Leu Ala Leu Trp Phe Met
       50                  55                  60
   Gly Leu Glu Asp His Pro Leu Arg Lys Arg Leu Gly Gln Ser Leu Leu
   65                  70                  75                  80
   Asp Ser Gln Arg Pro Asp Gly Ala Trp Gln Val Tyr Phe Gly Ala Pro
                   85                  90                  95
   Asn Gly Asp Ile Asn Ala Thr Val Glu Ala Tyr Ala Ala Leu Arg Ser
               100                 105                 110
   Leu Gly Phe Arg Asp Asp Glu Pro Ala Val Arg Arg Ala Arg Glu Trp
           115                 120                 125
   Ile Glu Ala Lys Gly Gly Leu Arg Asn Ile Arg Val Phe Thr Arg Tyr
       130                 135                 140
   Trp Leu Ala Leu Ile Gly Glu Trp Pro Trp Glu Lys Thr Pro Asn Ile
   145                 150                 155                 160
   Pro Pro Glu Val Ile Trp Phe Pro Leu Trp Phe Pro Phe Ser Ile Tyr
                   165                 170                 175
   Asn Phe Ala Gln Trp Ala Arg Ala Thr Leu Met Pro Ile Ala Val Leu
               180                 185                 190
   Ser Ala Arg Arg Pro Ser Arg Pro Leu Pro Pro Glu Asn Arg Leu Asp
           195                 200                 205
   Ala Leu Phe Pro His Gly Arg Lys Ala Phe Asp Tyr Glu Leu Pro Val
       210                 215                 220
   Lys Ala Gly Ala Gly Gly Trp Asp Arg Phe Phe Arg Gly Ala Asp Lys
   225                 230                 235                 240
   Val Leu His Lys Leu Gln Asn Leu Gly Asn Arg Leu Asn Leu Gly Leu
                   245                 250                 255
   Phe Arg Pro Ala Ala Thr Ser Arg Val Leu Glu Trp Met Ile Arg His
               260                 265                 270
   Gln Asp Phe Asp Gly Ala Trp Gly Gly Ile Gln Pro Pro Trp Ile Tyr
           275                 280                 285
   Gly Leu Met Ala Leu Tyr Ala Glu Gly Tyr Pro Leu Asn His Pro Val
       290                 295                 300
   Leu Ala Lys Gly Leu Asp Ala Leu Asn Asp Pro Gly Trp Arg Val Asp
   305                 310                 315                 320
   Val Gly Asp Ala Thr Tyr Ile Gln Ala Thr Asn Ser Pro Val Trp Asp
                   325                 330                 335
   Thr Ile Leu Thr Leu Leu Ala Phe Asp Asp Ala Gly Val Leu Gly Asp
               340                 345                 350
   Tyr Pro Glu Ala Val Asp Lys Ala Val Asp Trp Val Leu Gln Arg Gln
           355                 360                 365
   Val Arg Val Pro Gly Asp Trp Ser Met Lys Leu Pro His Val Lys Pro
       370                 375                 380
   Gly Gly Trp Ala Phe Glu Tyr Ala Asn Asn Tyr Tyr Pro Asp Thr Asp
   385                 390                 395                 400
   Asp Thr Ala Val Ala Leu Ile Ala Leu Ala Pro Leu Arg His Asp Pro
                   405                 410                 415
   Lys Trp Lys Ala Lys Gly Ile Asp Glu Ala Ile Gln Leu Gly Val Asp
               420                 425                 430
   Trp Leu Ile Gly Met Gln Ser Gln Gly Gly Gly Trp Gly Ala Phe Asp
           435                 440                 445
   Lys Asp Asn Asn Gln Lys Ile Leu Thr Lys Ile Pro Phe Cys Asp Tyr
       450                 455                 460
   Gly Glu Ala Leu Asp Pro Pro Ser Val Asp Val Thr Ala His Ile Ile
   465                 470                 475                 480
   Glu Ala Phe Gly Lys Leu Gly Ile Ser Arg Asn His Pro Ser Met Val
   485 490 495
   Gln Ala Leu Asp Tyr Ile Arg Arg Glu Gln Glu Pro Ser Gly Pro Trp
   500 505 510
   Phe Gly Arg Trp Gly Val Asn Tyr Val Tyr Gly Thr Gly Ala Val Leu
   515 520 525
   Pro Ala Leu Ala Ala Ile Gly Glu Asp Met Thr Gln Pro Tyr Ile Gly
   530 535 540
   Arg Ala Cys Asp Trp Leu Val Ala His Gln Gln Ala Asp Gly Gly Trp
   545 550 555 560
   Gly Glu Ser Cys Ala Ser Tyr Met Asp Val Ser Ala Val Gly Arg Gly
   565 570 575
   Thr Thr Thr Ala Ser Gln Thr Ala Trp Ala Leu Met Ala Leu Leu Ala
   580 585 590
   Ala Asn Arg Pro Gln Asp Lys Asp Ala Ile Glu Arg Gly Cys Met Trp
   595 600 605
   Leu Val Glu Arg Gln Ser Ala Gly Thr Trp Asp Glu Pro Glu Phe Thr
   610 615 620
   Gly Thr Gly Phe Pro Gly Tyr Gly Val Gly Gln Thr Ile Lys Leu Asn
   625 630 635 640
   Asp Pro Ala Leu Ser Gln Arg Leu Met Gln Gly Pro Glu Leu Ser Arg
   645 650 655
   Ala Phe Met Leu Arg Tyr Gly Met Tyr Arg His Tyr Phe Pro Leu Met
   660 665 670
   Ala Leu Gly Arg Ala Leu Arg Pro Gln Ser His Ser
   675 680
<210> 6
   <211> 657
   <212> PRT
   <213> Burkholderia ambifaria
<400> 6
   Met Asn Asp Leu Thr Glu Met Ala Thr Leu Ser Ala Gly Thr Val Pro
   1 5 10 15
   Ala Gly Leu Asp Ala Ala Val Ala Ser Ala Thr Asp Ala Leu Leu Ala
               20                  25                  30
   Ala Gln Asn Ala Asp Gly His Trp Val Tyr Glu Leu Glu Ala Asp Ser
           35                  40                  45
   Thr Ile Pro Ala Glu Tyr Val Leu Leu Val His Tyr Leu Gly Glu Thr
       50                  55                  60
   Pro Asn Leu Glu Leu Glu Gln Lys Ile Gly Arg Tyr Leu Arg Arg Val
   65                  70                  75                  80
   Gln Gln Ala Asp Gly Gly Trp Pro Leu Phe Thr Asp Gly Ala Pro Asn
                   85                  90                  95
   Ile Ser Ala Ser Val Lys Ala Tyr Phe Ala Leu Lys Val Ile Gly Asp
               100                 105                 110
   Asp Glu Asn Ala Glu His Met Gln Arg Ala Arg Arg Ala Ile Gln Ala
           115                 120                 125
   Met Gly Gly Ala Glu Met Ser Asn Val Phe Thr Arg Ile Gln Leu Ala
       130                 135                 140
   Leu Tyr Gly Ala Ile Pro Trp Arg Ala Val Pro Met Met Pro Val Glu
   145                 150                 155                 160
   Ile Met Leu Leu Pro Gln Trp Phe Pro Phe His Leu Ser Lys Val Ser
                   165                 170                 175
   Tyr Trp Ala Arg Thr Val Ile Val Pro Leu Leu Val Leu Asn Ala Lys
               180                 185                 190
   Arg Pro Ile Ala Lys Asn Pro Arg Gly Val Arg Ile Asp Glu Leu Phe
           195                 200                 205
   Val Asp Pro Pro Val Asn Ala Gly Leu Leu Pro Arg Gln Gly His Gln
       210                 215                 220
   Ser Pro Gly Trp Phe Ala Phe Phe Arg Val Val Asp His Ala Leu Arg
   225                 230                 235                 240
   Ala Ala Asp Gly Leu Phe Pro Asn Tyr Thr Arg Glu Arg Ala Ile Arg
                   245                 250                 255
   Gln Ala Val Ser Phe Val Asp Glu Arg Leu Asn Gly Glu Asp Gly Leu
               260                 265                 270
   Gly Ala Ile Tyr Pro Ala Met Ala Asn Ala Val Met Met Tyr Asp Val
           275                 280                 285
   Leu Gly Tyr Ala Glu Asp His Pro Asn Arg Ala Ile Ala Arg Lys Ser
       290                 295                 300
   Ile Glu Lys Leu Leu Val Val Gln Glu Asp Glu Ala Tyr Cys Gln Pro
   305                 310                 315                 320
   Cys Leu Ser Pro Val Trp Asp Thr Ser Leu Ala Ala His Ala Leu Leu
                   325                 330                 335
   Glu Thr Gly Asp Ala Arg Ala Glu Glu Ala Val Ile Arg Gly Leu Glu
               340                 345                 350
   Trp Leu Arg Pro Leu Gln Ile Leu Asp Val Arg Gly Asp Trp Ile Ser
           355                 360                 365
   Arg Arg Pro His Val Arg Pro Gly Gly Trp Ala Phe Gln Tyr Ala Asn
       370                 375                 380
   Pro His Tyr Pro Asp Val Asp Asp Thr Ala Val Val Ala Val Ala Met
   385                 390                 395                 400
   Asp Arg Val Gln Lys Leu Lys His Asn Asp Ala Phe Arg Asp Ser Ile
                   405                 410                 415
   Ala Arg Ala Arg Glu Trp Val Val Gly Met Gln Ser Ser Asp Gly Gly
               420                 425                 430
   Trp Gly Ala Phe Glu Pro Glu Asn Thr Gln Tyr Tyr Leu Asn Asn Ile
           435                 440                 445
   Pro Phe Ser Asp His Gly Ala Leu Leu Asp Pro Pro Thr Ala Asp Val
       450                 455                 460
   Ser Gly Arg Cys Leu Ser Met Leu Ala Gln Leu Gly Glu Thr Pro Leu
   465                 470                 475                 480
   Asn Ser Glu Pro Ala Arg Arg Ala Leu Asp Tyr Met Leu Lys Glu Gln
                   485                 490                 495
   Glu Pro Asp Gly Ser Trp Tyr Gly Arg Trp Gly Met Asn Tyr Val Tyr
               500                 505                 510
   Gly Thr Trp Thr Ala Leu Cys Ala Leu Asn Ala Ala Gly Leu Thr Pro
                   515                 520                 525
   Asp Asp Pro Arg Val Lys Arg Gly Ala Gln Trp Leu Leu Ser Ile Gln
       530                 535                 540
   Asn Lys Asp Gly Gly Trp Gly Glu Asp Gly Asp Ser Tyr Lys Leu Asn
   545                 550                 555                 560
   Tyr Arg Gly Phe Glu Gln Ala Pro Ser Thr Ala Ser Gln Thr Ala Trp
                   565                 570                 575
   Ala Leu Leu Gly Leu Met Ala Ala Gly Glu Val Asn Asn Pro Ala Val
               580                 585                 590
   Ala Arg Gly Val Glu Tyr Leu Ile Ala Glu Gln Lys Glu His Gly Leu
           595                 600                 605
   Trp Asp Glu Thr Arg Phe Thr Ala Thr Gly Phe Pro Arg Val Phe Tyr
       610                 615                 620
   Leu Arg Tyr His Gly Tyr Arg Lys Phe Phe Pro Leu Trp Ala Leu Ala
   625                 630                 635                 640
   Arg Tyr Arg Asn Leu Lys Arg Asn Asn Ala Thr Arg Val Thr Phe Gly
                   645                 650                 655
   Leu
<210> 7
   <211> 682
   <212> PRT
   <213> Burkholderia ambifaria
<400> 7
   Met Ile Arg Arg Met Asn Lys Ser Gly Pro Ser Pro Trp Ser Ala Leu
   1               5                   10                  15
   Asp Ala Ala Ile Ala Arg Gly Arg Asp Ala Leu Met Arg Leu Gln Gln
               20                  25                  30
   Pro Asp Gly Ser Trp Cys Phe Glu Leu Glu Ser Asp Ala Thr Ile Thr
           35                  40                  45
   Ala Glu Tyr Ile Leu Met Met His Phe Met Asp Lys Ile Asp Asp Ala
       50                  55                  60
   Arg Gln Glu Lys Met Ala Arg Tyr Leu Arg Ala Ile Gln Arg Leu Asp
   65 70 75 80
   Thr His Gly Gly Trp Asp Leu Tyr Val Asp Gly Asp Pro Asp Val Ser
   85 90 95
   Cys Ser Val Lys Ala Tyr Phe Ala Leu Lys Ala Ala Gly Asp Ser Glu
   100 105 110
   His Ala Pro His Met Val Arg Ala Arg Asp Ala Ile Leu Glu Leu Gly
   115 120 125
   Gly Ala Ala Arg Ser Asn Val Phe Thr Arg Ile Leu Leu Ala Thr Phe
   130 135 140
   Gly Gln Val Pro Trp Arg Ala Thr Pro Phe Met Pro Ile Glu Phe Val
   145 150 155 160
   Leu Phe Pro Lys Trp Val Pro Ile Ser Met Tyr Lys Val Ala Tyr Trp
   165 170 175
   Ala Arg Thr Thr Met Val Pro Leu Leu Val Leu Cys Ser Leu Lys Ala
   180 185 190
   Arg Ala Arg Asn Pro Arg Asn Ile Ala Ile Pro Glu Leu Phe Val Thr
   195 200 205
   Pro Pro Asp Gln Glu Arg Gln Tyr Phe Pro Pro Ala Arg Gly Met Arg
   210 215 220
   Arg Ala Phe Leu Ala Leu Asp Arg Val Val Arg His Val Glu Pro Leu
   225 230 235 240
   Leu Pro Lys Arg Leu Arg Gln Arg Ala Ile Arg His Ala Gln Ala Trp
   245 250 255
   Cys Ala Glu Arg Met Asn Gly Glu Asp Gly Leu Gly Gly Ile Phe Pro
   260 265 270
   Pro Ile Val Tyr Ser Tyr Gln Met Met Asp Val Leu Gly Tyr Pro Asp
   275 280 285
   Asp His Pro Leu Arg Arg Asp Cys Glu Asn Ala Leu Glu Lys Leu Leu
   290 295 300
   Val Thr Arg Pro Asp Gly Ser Met Tyr Cys Gln Pro Cys Leu Ser Pro
   305 310 315 320
   Val Trp Asp Thr Ala Trp Ser Thr Met Ala Leu Glu Gln Ala Arg Gly
                   325                 330                 335
   Val Ala Val Pro Glu Ala Gly Ala Pro Ala Ser Ala Leu Asp Glu Leu
               340                 345                 350
   Asp Ala Arg Ile Ala Arg Ala Tyr Asp Trp Leu Ala Glu Arg Gln Val
           355                 360                 365
   Asn Asp Leu Arg Gly Asp Trp Ile Glu Asn Ala Pro Ala Asp Thr Gln
       370                 375                 380
   Pro Gly Gly Trp Ala Phe Gln Tyr Ala Asn Pro Tyr Tyr Pro Asp Ile
   385                 390                 395                 400
   Asp Asp Ser Ala Val Val Thr Ala Met Leu Asp Arg Arg Gly Arg Thr
                   405                 410                 415
   His Arg Asn Ala Asp Gly Ser His Pro Tyr Ala Ala Arg Val Ala Arg
               420                 425                 430
   Ala Leu Asp Trp Met Arg Gly Leu Gln Ser Arg Asn Gly Gly Phe Ala
           435                 440                 445
   Ala Phe Asp Ala Asp Cys Asp Arg Leu Tyr Leu Asn Ala Ile Pro Phe
       450                 455                 460
   Ala Asp His Gly Ala Leu Leu Asp Pro Pro Thr Glu Asp Val Ser Gly
   465                 470                 475                 480
   Arg Val Leu Leu Cys Phe Gly Val Thr Lys Arg Ala Asp Asp Arg Ala
                   485                 490                 495
   Ser Leu Ala Arg Ala Ile Asp Tyr Val Lys Arg Thr Gln Gln Pro Asp
               500                 505                 510
   Gly Ser Trp Trp Gly Arg Trp Gly Thr Asn Tyr Leu Tyr Gly Thr Trp
           515                 520                 525
   Ser Val Leu Ala Gly Leu Ala Leu Ala Gly Glu Asp Pro Ser Gln Pro
       530                 535                 540
   Tyr Ile Ala Arg Ala Leu Ala Trp Leu Arg Ala Arg Gln His Ala Asp
   545                 550                 555                 560
   Gly Gly Trp Gly Glu Thr Asn Asp Ser Tyr Ile Asp Pro Ala Leu Ala
                   565                 570                 575
   Gly Thr Asn Ala Gly Glu Ser Thr Ser Asn Cys Thr Ala Trp Ala Leu
   580 585 590
   Leu Ala Gln Met Ala Phe Gly Asp Gly Glu Ser Glu Ser Val Arg Arg
   595 600 605
   Gly Ile Ala Tyr Leu Gln Ser Val Gln Gln Asp Asp Gly Phe Trp Trp
   610 615 620
   His Arg Ser His Asn Ala Pro Gly Phe Pro Arg Ile Phe Tyr Leu Lys
   625 630 635 640
   Tyr His Gly Tyr Thr Ala Tyr Phe Pro Leu Trp Ala Leu Ala Arg Tyr
   645 650 655
   Arg Arg Leu Ala Gly Gly Val Ser Ala Ala Gly Ala His Ala Val Pro
   660 665 670
   Ala Ser Thr Gly Ala Asp Ala Ala Leu Ala
   675 680
<210> 8
   <211> 617
   <212> PRT
   <213> Bacillus anthracis
<400> 8
   Met Leu Leu Tyr Glu Lys Ala His Glu Glu Ile Val Arg Arg Ala Thr
   1 5 10 15
   Ala Leu Gln Thr Met Gln Trp Gln Asp Gly Thr Trp Arg Phe Cys Phe
   20 25 30
   Glu Gly Ala Pro Leu Thr Asp Cys His Met Ile Phe Leu Leu Lys Leu
   35 40 45
   Leu Gly Arg Asp Lys Glu Ile Glu Pro Phe Val Glu Arg Val Ala Ser
   50 55 60
   Leu Gln Thr Asn Glu Gly Thr Trp Lys Leu His Glu Asp Glu Val Gly
   65 70 75 80
   Gly Asn Leu Ser Ala Thr Ile Gln Ser Tyr Ala Ala Leu Leu Ala Ser
   85 90 95
   Lys Lys Tyr Thr Lys Glu Asp Ala Asn Met Lys Arg Ala Glu Asn Phe
   100 105 110
   Ile Gln Glu Arg Gly Gly Val Ala Arg Ala His Phe Met Thr Lys Phe
           115                 120                 125
   Leu Leu Ala Ile His Gly Glu Tyr Glu Tyr Pro Ser Leu Phe His Leu
       130                 135                 140
   Pro Thr Pro Ile Met Phe Leu Gln Asn Asp Ser Pro Phe Ser Ile Phe
   145                 150                 155                 160
   Glu Leu Ser Ser Ser Ala Arg Ile His Leu Ile Pro Met Met Leu Cys
                   165                 170                 175
   Leu Asn Lys Arg Phe Arg Val Gly Lys Lys Leu Leu Pro Asn Leu Asn
               180                 185                 190
   His Ile Ala Gly Gly Gly Gly Glu Trp Phe Arg Glu Asp Arg Ser Pro
           195                 200                 205
   Val Phe Gln Thr Leu Leu Ser Asp Val Lys Gln Ile Ile Ser Tyr Pro
       210                 215                 220
   Leu Ser Leu His His Lys Gly Tyr Glu Glu Ile Glu Arg Phe Met Lys
   225                 230                 235                 240
   Glu Arg Ile Asp Glu Asn Gly Thr Leu Tyr Ser Tyr Ala Thr Ala Ser
                   245                 250                 255
   Phe Tyr Met Ile Tyr Ala Leu Leu Ala Leu Gly His Ser Leu Gln Ser
               260                 265                 270
   Ser Met Ile Gln Lys Ala Ile Ala Gly Ile Thr Ser Tyr Ile Trp Lys
           275                 280                 285
   Met Glu Arg Gly Asn His Leu Gln Asn Ser Pro Ser Thr Val Trp Asp
       290                 295                 300
   Thr Ala Leu Leu Ser Tyr Ala Leu Gln Glu Ala Gln Val Ser Lys Asp
   305                 310                 315                 320
   Asn Lys Met Ile Gln Asn Ala Thr Ala Tyr Leu Leu Lys Lys Gln His
                   325                 330                 335
   Thr Lys Lys Ala Asp Trp Ser Val His Ala Pro Ala Leu Thr Pro Gly
               340                 345                 350
   Gly Trp Gly Phe Ser Asp Val Asn Thr Thr Ile Pro Asp Ile Asp Asp
           355                 360                 365
   Thr Thr Ala Val Leu Arg Ala Leu Ala Arg Ser Arg Gly Asn Lys Asn
       370                 375                 380
   Ile Asp Asn Ala Trp Lys Lys Gly Gly Asn Trp Ile Lys Gly Leu Gln
   385                 390                 395                 400
   Asn Asn Asp Gly Gly Trp Gly Ala Phe Glu Lys Gly Val Thr Ser Lys
                   405                 410                 415
   Leu Leu Ala Lys Leu Pro Ile Glu Asn Ala Ser Asp Met Ile Thr Asp
               420                 425                 430
   Pro Ser Thr Pro Asp Ile Thr Gly Arg Val Leu Glu Phe Phe Gly Thr
           435                 440                 445
   Tyr Ala Gln Asn Glu Leu Pro Glu Lys Gln Ile Gln Arg Ala Ile Asn
       450                 455                 460
   Trp Leu Met Asn Val Gln Glu Glu Asn Gly Ser Trp Tyr Gly Lys Trp
   465                 470                 475                 480
   Gly Ile Cys Tyr Leu Tyr Gly Thr Trp Ala Val Met Thr Gly Leu Arg
                   485                 490                 495
   Ser Leu Gly Ile Pro Ser Ser Asn Pro Ser Leu Thr Arg Ala Ala Ser
               500                 505                 510
   Trp Leu Glu His Ile Gln His Glu Asp Gly Gly Trp Gly Glu Ser Cys
           515                 520                 525
   His Ser Ser Val Glu Lys Arg Phe Val Thr Leu Pro Phe Ser Thr Pro
       530                 535                 540
   Ser Gln Thr Ala Trp Ala Leu Asp Ala Leu Ile Ser Tyr Tyr Asp Thr
   545                 550                 555                 560
   Glu Thr Pro Ala Ile Arg Lys Gly Val Ser Tyr Leu Leu Ser Asn Pro
                   565                 570                 575
   Tyr Val Asn Glu Arg Tyr Pro Thr Gly Thr Gly Leu Pro Gly Ala Phe
               580                 585                 590
   Tyr Ile Arg Tyr His Ser Tyr Ala His Ile Tyr Pro Leu Leu Thr Leu
           595                 600                 605
   Ala His Tyr Ile Lys Lys Tyr Arg Lys
   610 615
<210> 9
   <211> 720
   <212> PRT
   <213> Frankia alni
<400> 9
   Met Pro Ala Gly Val Gly Val Leu Val Trp Leu Asp Gln Arg Leu Arg
   1               5                   10                  15
   Ala Met Gly Arg Pro Asp Leu Val Thr Thr Thr Gly Gly Ala Glu Ile
               20                  25                  30
   Pro Phe Val Leu Val Ala Ala Thr Ala Ser Thr Val Gly Val Ala Leu
           35                  40                  45
   Ala Leu Arg Arg Pro Arg His Pro Val Gly Trp Leu Phe Leu Ala Leu
       50                  55                  60
   Gly Gly Val Leu Leu Leu Ser Gly Gly Thr Gln Gly Tyr Ala Ala Tyr
   65                  70                  75                  80
   Gly Ala Val Ala Arg Pro Gly Arg Leu Pro Ala Ala Asp Leu Val Ala
                   85                  90                  95
   Ile Tyr Ala Asp Ala Gly Phe Ile Pro Trp Leu Val Leu Val Ala Leu
               100                 105                 110
   Ile Leu His Leu Thr Pro Thr Gly Arg Pro Leu Ser Ala Arg Trp Gly
           115                 120                 125
   Arg Ile Ala Leu Ala Thr Ala Val Ala Gly Gly Leu Trp Leu Leu Val
       130                 135                 140
   Gly Leu Val Thr Thr Glu Thr Met Gln Pro Pro Phe Gln Ser Val Thr
   145                 150                 155                 160
   Asn Pro Leu Leu Ile Gly Gly Pro Leu Gly Pro Leu Leu Val Ala Arg
                   165                 170                 175
   Arg Val Leu Gly Leu Ala Thr Gly Ala Gly Val Val Leu Ala Ala Val
               180                 185                 190
   Ser Leu Ile Val Arg Phe Arg Arg Ser Val Asp Val Glu Arg Arg Gln
           195                 200                 205
   Leu Leu Trp Val Ala Val Ala Ala Val Pro Leu Pro Val Leu Met Ala
       210                 215                 220
   Ala Ser Phe Ala Ala Ser Tyr Ala Gly Asn Asn Thr Ala Ala Gly Leu
   225                 230                 235                 240
   Ala Ala Ala Thr Leu Ile Gly Leu Leu Ala Ile Gly Ala Gly Leu Ala
                   245                 250                 255
   Ile Gly Gln Tyr His Leu Tyr Asp Val Glu Glu Ile Leu Ser Arg Ala
               260                 265                 270
   Val Thr Tyr Leu Leu Val Ser Gly Leu Leu Ala Ala Ser Tyr Ala Thr
           275                 280                 285
   Val Val Ile Val Val Gly Gln Ser Leu Ala Gly Arg Thr Gly Arg Ser
       290                 295                 300
   Gln Ile Ser Ala Val Leu Ala Thr Leu Ala Ala Val Ala Val Thr Ala
   305                 310                 315                 320
   Pro Ala Tyr Arg Lys Ile Gln Glu Gly Val Asp Arg Arg Phe Ser Arg
                   325                 330                 335
   Arg Arg Phe Glu Thr Leu Gln Val Ile Arg Arg Tyr Leu Arg Asp Pro
               340                 345                 350
   Asp Pro Asp Val Ala Val Glu Glu Val Leu Arg Arg Ala Leu Gly Asp
           355                 360                 365
   Pro Thr Leu Ala Val Ala Tyr Leu Val Asp Asp Arg Arg Gln Trp Val
       370                 375                 380
   Ser Ala Asp Gly Gln Pro Ala Asn Pro Gly Asn Ser Phe Met Ala Ala
   385                 390                 395                 400
   Val Glu Val Tyr Arg Arg Gly Arg Pro Ile Ala Arg Val Thr Phe Asp
                   405                 410                 415
   Arg Gly Arg Ala Gln Pro Gly Leu Val Arg Ala Ala Ala Thr Ala Ala
               420                 425                 430
   Thr Ala Glu Leu Asp Asn Ala Gly Leu Arg Ala Ala Val Ala Leu Gln
           435                 440                 445
   Leu Val Glu Val Arg Gln Ser Arg Thr Arg Ile Ala Ala Ala Gln Phe
       450                 455                 460
   Ala Glu Arg Arg Thr Ile Glu Arg Asn Leu His Asp Gly Ala Gln Gln
   465                 470                 475                 480
   Arg Leu Leu Ala Leu Ala Leu Gln Leu Arg Ala Val Gln Leu Gly Gly
                   485                 490                 495
   Asp Glu Ala Ser Leu Arg Gln Ala Ile Ser Thr Gly Ile Asp Gln Leu
               500                 505                 510
   Gln Ala Ala Val Val Glu Leu Arg Glu Leu Ala Asn Gly Leu His Pro
           515                 520                 525
   Ala Val Leu Ala Asp Gly Gly Leu Ala Ala Ala Leu Asp Asp Val Ala
       530                 535                 540
   Ala Arg Thr Pro Val Pro Ile Lys Ile Ser Ala Pro Asp Arg Arg Tyr
   545                 550                 555                 560
   Pro Pro Asp Leu Glu Ala Ala Ala Trp Phe Ile Ala Cys Glu Ala Met
                   565                 570                 575
   Ala Asn Ala Val Lys His Ala His Pro Thr Thr Ile Ala Val Asp Val
               580                 585                 590
   Ser Ala Pro Asp Gly Gln Leu Ile Val Glu Val Arg Asp Asp Gly Ile
           595                 600                 605
   Gly Gly Ala Gln Pro Ser Gly Pro Gly Leu Arg Gly Ile Ala Asp Arg
       610                 615                 620
   Ala Glu Ala Phe Gly Gly Ser Leu Thr Val His Thr Asp Pro Gly Thr
   625                 630                 635                 640
   Gly Thr Thr Ile Arg Ala Leu Leu His Arg Arg Ser Pro Leu Ser Ser
                   645                 650                 655
   Gly Arg Arg Ser Val Met Ile Glu Gly Cys Val Asp Val Val Ala Val
               660                 665                 670
   Arg Arg Phe Arg Cys Arg Ser Ser Arg Gly Ser Gly Ser Arg Arg Arg
           675                 680                 685
   Arg Ser Ser Trp Arg Cys Gly Gly Ile Cys Gly Ser Arg Cys Arg Thr
       690                 695                 700
   Gly Met Ser Arg Ser Cys Ser Arg Asn Ala Ala Ser Lys Leu Ile Thr
   705                 710                 715                 720
<210> 10
   <211> 685
   <212> PRT
   <213> Rhodopseudomonas palent
<400> 10
   Met Asp Ser Ile Leu Ala Pro Arg Ala Asp Ala Pro Arg Asn Ile Asp
   1               5                   10                  15
   Gly Ala Leu Arg Glu Ser Val Gln Gln Ala Ala Asp Trp Leu Val Ala
               20                  25                  30
   Asn Gln Lys Pro Asp Gly His Trp Val Gly Arg Ala Glu Thr Asn Ala
           35                  40                  45
   Thr Met Glu Ala Gln Trp Cys Leu Ala Leu Trp Phe Leu Gly Leu Glu
       50                  55                  60
   Asp His Pro Leu Arg Val Arg Leu Gly Arg Ala Leu Leu Asp Thr Gln
   65                  70                  75                  80
   Arg Pro Asp Gly Ala Trp His Val Phe Tyr Gly Ala Pro Asn Gly Asp
                   85                  90                  95
   Ile Asn Ala Thr Val Glu Ala Tyr Ala Ala Leu Arg Ser Leu Gly His
               100                 105                 110
   Arg Asp Asp Glu Glu Pro Leu Arg Lys Ala Arg Asp Trp Ile Leu Ser
           115                 120                 125
   Lys Gly Gly Leu Ala Asn Ile Arg Val Phe Thr Arg Tyr Trp Leu Ala
       130                 135                 140
   Leu Ile Gly Glu Trp Pro Trp Glu Lys Thr Pro Asn Ile Leu Pro Glu
   145                 150                 155                 160
   Val Ile Trp Leu Pro Thr Trp Phe Pro Phe Ser Ile Tyr Asn Phe Ala
                   165                 170                 175
   Gln Trp Ala Arg Ala Thr Leu Met Pro Ile Ala Val Leu Ser Ala His
               180                 185                 190
   Arg Pro Ser Arg Pro Leu Ala Pro Gln Asp Arg Leu Asp Ala Leu Phe
           195                 200                 205
   Pro Gln Gly Arg Asp Ser Phe Asn Tyr Asp Leu Pro Ala Arg Leu Gly
       210                 215                 220
   Ala Gly Val Trp Asp Val Ile Phe Arg Lys Ile Asp Thr Ile Leu His
   225                 230                 235                 240
   Arg Leu Gln Asp Trp Gly Ala Arg Arg Gly Pro His Gly Ile Met Arg
                   245                 250                 255
   Arg Gly Ala Ile Asp His Val Leu Gln Trp Ile Ile Arg His Gln Asp
               260                 265                 270
   Tyr Asp Gly Ser Trp Gly Gly Ile Gln Pro Pro Trp Ile Tyr Gly Leu
           275                 280                 285
   Met Ala Leu His Thr Glu Gly Tyr Ala Met Thr His Pro Val Met Ala
       290                 295                 300
   Lys Ala Leu Asp Ala Leu Asn Glu Pro Gly Trp Arg Ile Asp Ile Gly
   305                 310                 315                 320
   Asp Ala Thr Phe Ile Gln Ala Thr Asn Ser Pro Val Trp Asp Thr Met
                   325                 330                 335
   Leu Ser Leu Leu Ala Phe Asp Asp Ala Gly Leu Gly Glu Arg Tyr Pro
               340                 345                 350
   Glu Gln Val Glu Arg Ala Val Arg Trp Val Leu Lys Arg Gln Val Leu
           355                 360                 365
   Val Pro Gly Asp Trp Ser Val Lys Leu Pro Asp Val Lys Pro Gly Gly
       370                 375                 380
   Trp Ala Phe Glu Tyr Ala Asn Asn Phe Tyr Pro Asp Thr Asp Asp Thr
   385                 390                 395                 400
   Ser Val Ala Leu Met Ala Leu Ala Pro Phe Arg His Asp Pro Lys Trp
                   405                 410                 415
   Gln Ala Glu Gly Ile Glu Asp Ala Ile Gln Arg Gly Ile Asp Trp Leu
               420                 425                 430
   Val Ala Met Gln Cys Lys Glu Gly Gly Trp Gly Ala Phe Asp Lys Asp
           435                 440                 445
   Asn Asp Lys Lys Ile Leu Ala Lys Ile Pro Phe Cys Asp Phe Gly Glu
       450                 455                 460
   Ala Leu Asp Pro Pro Ser Ala Asp Val Thr Ala His Ile Ile Glu Ala
   465                 470                 475                 480
   Phe Ala Lys Val Gly Leu Asp Arg Asn His Pro Ser Ile Val Arg Ala
   485 490 495
   Leu Asp Tyr Leu Lys Arg Glu Gln Glu Pro Glu Gly Pro Trp Phe Gly
   500 505 510
   Arg Trp Gly Val Asn Tyr Val Tyr Gly Thr Gly Ala Val Leu Pro Ala
   515 520 525
   Leu Ala Ala Ile Gly Glu Asp Met Arg Gln Pro Tyr Ile Ala Arg Ala
   530 535 540
   Cys Asp Trp Leu Ile Ala Arg Gln Gln Ala Asn Gly Gly Trp Gly Glu
   545 550 555 560
   Ser Cys Val Ser Tyr Met Asp Ala Lys Gln Ala Gly Glu Gly Thr Ala
   565 570 575
   Thr Ala Ser Gln Thr Ala Trp Ala Leu Met Ala Leu Ile Ala Ala Asp
   580 585 590
   Arg Pro Gln Asp Arg Asp Ala Ile Glu Arg Gly Cys Leu Tyr Leu Thr
   595 600 605
   Glu Thr Gln Arg Asp Gly Thr Trp Gln Glu Val His Tyr Thr Gly Thr
   610 615 620
   Gly Phe Pro Gly Tyr Gly Val Gly Gln Thr Ile Lys Leu Asn Asp Pro
   625 630 635 640
   Leu Leu Ser Lys Arg Leu Met Gln Gly Pro Glu Leu Ser Arg Ser Phe
   645 650 655
   Met Leu Arg Tyr Asp Leu Tyr Arg His Tyr Phe Pro Met Met Ala Ile
   660 665 670
   Gly Arg Val Leu Arg Gln Arg Gly Asp Arg Ser Gly His
   675 680 685
<210> 11
   <211> 680
   <212> PRT
   <213> Streptomyces coelicolor
<400> 11
   Met Thr Ala Thr Thr Asp Gly Ser Thr Gly Ala Ser Leu Arg Pro Leu
   1 5 10 15
   Ala Ala Ser Ala Ser Asp Thr Asp Ile Thr Ile Pro Ala Ala Ala Ala
               20                  25                  30
   Gly Val Pro Glu Ala Ala Ala Arg Ala Thr Arg Arg Ala Thr Asp Phe
           35                  40                  45
   Leu Leu Ala Lys Gln Asp Ala Glu Gly Trp Trp Lys Gly Asp Leu Glu
       50                  55                  60
   Thr Asn Val Thr Met Asp Ala Glu Asp Leu Leu Leu Arg Gln Phe Leu
   65                  70                  75                  80
   Gly Ile Gln Asp Glu Glu Thr Thr Arg Ala Ala Ala Leu Phe Ile Arg
                   85                  90                  95
   Gly Glu Gln Arg Glu Asp Gly Thr Trp Ala Thr Phe Tyr Gly Gly Pro
               100                 105                 110
   Gly Glu Leu Ser Thr Thr Ile Glu Ala Tyr Val Ala Leu Arg Leu Ala
           115                 120                 125
   Gly Asp Ser Pro Glu Ala Pro His Met Ala Arg Ala Ala Glu Trp Ile
       130                 135                 140
   Arg Ser Arg Gly Gly Ile Ala Ser Ala Arg Val Phe Thr Arg Ile Trp
   145                 150                 155                 160
   Leu Ala Leu Phe Gly Trp Trp Lys Trp Asp Asp Leu Pro Glu Leu Pro
                   165                 170                 175
   Pro Glu Leu Ile Tyr Phe Pro Thr Trp Val Pro Leu Asn Ile Tyr Asp
               180                 185                 190
   Phe Gly Cys Trp Ala Arg Gln Thr Ile Val Pro Leu Thr Ile Val Ser
           195                 200                 205
   Ala Lys Arg Pro Val Arg Pro Ala Pro Phe Pro Leu Asp Glu Leu His
       210                 215                 220
   Thr Asp Pro Ala Arg Pro Asn Pro Pro Arg Pro Leu Ala Pro Val Ala
   225                 230                 235                 240
   Ser Trp Asp Gly Ala Phe Gln Arg Ile Asp Lys Ala Leu His Ala Tyr
                   245                 250                 255
   Arg Lys Val Ala Pro Arg Arg Leu Arg Arg Ala Ala Met Asn Ser Ala
   260 265 270
   Ala Arg Trp Ile Ile Glu Arg Gln Glu Asn Asp Gly Cys Trp Gly Gly
   275 280 285
   Ile Gln Pro Pro Ala Val Tyr Ser Val Ile Ala Leu Tyr Leu Leu Gly
   290 295 300
   Tyr Asp Leu Glu His Pro Val Met Arg Ala Gly Leu Glu Ser Leu Asp
   305 310 315 320
   Arg Phe Ala Val Trp Arg Glu Asp Gly Ala Arg Met Ile Glu Ala Cys
   325 330 335
   Gln Ser Pro Val Trp Asp Thr Cys Leu Ala Thr Ile Ala Leu Ala Asp
   340 345 350
   Ala Gly Val Pro Glu Asp His Pro Gln Leu Val Lys Ala Ser Asp Trp
   355 360 365
   Met Leu Gly Glu Gln Ile Val Arg Pro Gly Asp Trp Ser Val Lys Arg
   370 375 380
   Pro Gly Leu Pro Pro Gly Gly Trp Ala Phe Glu Phe His Asn Asp Asn
   385 390 395 400
   Tyr Pro Asp Ile Asp Asp Thr Ala Glu Val Val Leu Ala Leu Arg Arg
   405 410 415
   Val Arg His His Asp Pro Glu Arg Val Glu Lys Ala Ile Gly Arg Gly
   420 425 430
   Val Arg Trp Asn Leu Gly Met Gln Ser Lys Asn Gly Ala Trp Gly Ala
   435 440 445
   Phe Asp Val Asp Asn Thr Ser Ala Phe Pro Asn Arg Leu Pro Phe Cys
   450 455 460
   Asp Phe Gly Glu Val Ile Asp Pro Pro Ser Ala Asp Val Thr Ala His
   465 470 475 480
   Val Val Glu Met Leu Ala Val Glu Gly Leu Ala His Asp Pro Arg Thr
   485 490 495
   Arg Arg Gly Ile Gln Trp Leu Leu Asp Ala Gln Glu Thr Asp Gly Ser
   500 505 510
   Trp Phe Gly Arg Trp Gly Val Asn Tyr Val Tyr Gly Thr Gly Ser Val
           515                 520                 525
   Ile Pro Ala Leu Thr Ala Ala Gly Leu Pro Thr Ser His Pro Ala Ile
       530                 535                 540
   Arg Arg Ala Val Arg Trp Leu Glu Ser Val Gln Asn Glu Asp Gly Gly
   545                 550                 555                 560
   Trp Gly Glu Asp Leu Arg Ser Tyr Arg Tyr Val Arg Glu Trp Ser Gly
                   565                 570                 575
   Arg Gly Ala Ser Thr Ala Ser Gln Thr Gly Trp Ala Leu Met Ala Leu
               580                 585                 590
   Leu Ala Ala Gly Glu Arg Asp Ser Lys Ala Val Glu Arg Gly Val Ala
           595                 600                 605
   Trp Leu Ala Ala Thr Gln Arg Glu Asp Gly Ser Trp Asp Glu Pro Tyr
       610                 615                 620
   Phe Thr Gly Thr Gly Phe Pro Trp Asp Phe Ser Ile Asn Tyr Asn Leu
   625                 630                 635                 640
   Tyr Arg Gln Val Phe Pro Leu Thr Ala Leu Gly Arg Tyr Val His Gly
                   645                 650                 655
   Glu Pro Phe Ala Lys Lys Pro Arg Ala Ala Asp Ala Pro Ala Glu Ala
               660                 665                 670
   Ala Pro Ala Glu Val Lys Gly Ser
           675                 680

## Beschreibung

Die Erfindung betrifft ein Verfahren zur biokatalytischen Herstellung von Ambroxan.

Verbindungen mit dem Grundgerüst des Dodecahydronaphtho[2,1-b]furans sind als Aromachemikalien von großem wirtschaftlichem Interesse. Hier ist Verbindung 2 zu nennen, das als das linksdrehende Stereoisomer [(-)-2] des Ambroxan bekannte (3a*R*,5a*S*,9a*S*,9b*R*)-3a,6,6,9a-Tetramethyldodecahydronaphtho-[2,1-*b*]-furan).

Ursprünglich aus dem Ambra des Pottwals gewonnen, gibt es heute vor allem zwei Routen, die Zugang zu Ambroxan erlauben. Sclareol (3), ein Inhaltsstoff des Muskatellersalbeis (*Salvia sclarea*) dient dabei oft als geeignetes Ausgangsmaterial für semisynthetisches Material, weil es die optische Information für Verbindung ((-)-2) bereits enthält. Dabei kann man den oxidativen Abbau mit Chromsäure, Permanganat, H2O2 oder Ozon durchführen [Stoll et al.; Helv Chim Acta (1950), 33: 1251]. Das erhaltene Sclareolid (4) wird durch eine anschließende Reduktion (z.B. mit LiAIH4 oder NaBH4) in das Ambrox-1,4-diol (5) überführt [Mookherjee et al.; Perfumer and Flavourist (1990), 15: 27]. Die Herstellung von Verbindung (4) aus Sclareol (3) kann auch durch eine Biotransformation mit *Hyphozyma roseoniger* erfolgen[EP 204009].

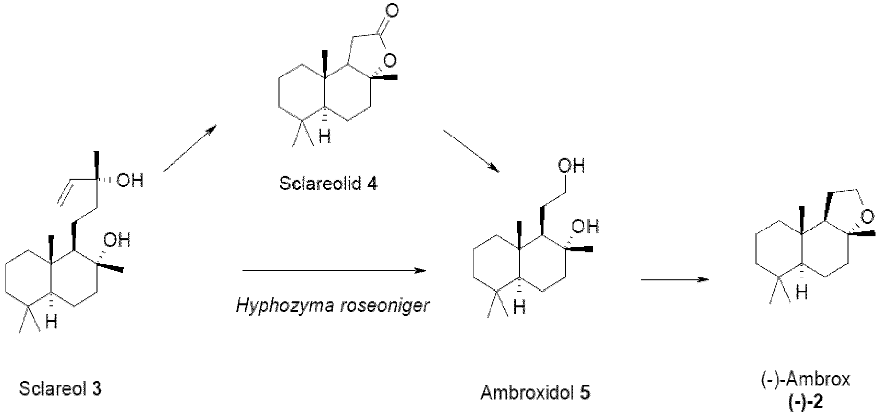

Ambrox-1,4-diol (5) kann schließlich mit einer Reihe von chemischen Verfahren zu Verbindung ((-)-2) cyclisiert werden. Zu dem Racemat von Ambroxan rac-2 sind Zugänge u.a. via Homofarnesylsäure [US 513,270; Lucius et al.; Chem Ber (1960), 93: 2663] und 4-(2,6,6-Trimethyl-cyclohex-1-enyl)-butan-2-on [Büchi et al.; Helv Chim Acta (1989), 72: 996] bearbeitet worden. Das Marktvolumen von Ambroxan lag 2002 zurzeit mittelwertig bei 20 Tonnen pro Jahr. Hierfür ist eine Ausgangsbasis von ca. 33 Tonnen Sclareol pro Jahr notwendig. Für die Produktion einer Tonne Ambroxan werden 207 Tonnen verschiedener Einzelstoffe benötigt, die wiederum den Anfall von 206 Tonnen Abfall bedingen. Die anfallenden Substanzen weisen unterschiedliche, insgesamt aber verhältnismäßig starke Umwelt- und Gesundheitswirkungen auf [Deutsche Bundesstiftung Umwelt]. Somit ist diese Synthese sehr energieaufwändig und erfordert den Einsatz umweltbelastender Chemikalien.

Die biokatalytische Synthese von Verbindung ((-)-2) ist in der Literatur beschrieben [Neumann et al.; Biol Chem Hoppe Seyler (1986), 367: 723]. Dabei wird das Molekül aus Homofarnesol (Verbindung (1), (3*Z*,7*E*)-4,8,12-Trimethyltrideca-3,7,11-trien-1-ol) gewonnen. Als Katalysator wurde die Squalen-Hopen-Cyclase (SHC) aus *Alicyclobacillus acidocaldarius* (ehem. *Bacillus acidocaldarius*) verwendet. Das Enzym katalysiert natürlicherweise die Zyklisierung von Squalen zu Hopan. Als Nebenreaktion kann diese SHC offenbar auch Verbindung (1) zu Ambroxan ((-)-2) umsetzen. Der Biokatalysator kann rekombinant hergestellt werden [Ochs D.et al.; J Bacteriol (1992), 174: 298]. Der Umsatz der Cyclisierung von Homofarnesol zu Ambroxan beträgt nach Neumann et al. allerdings lediglich 1,2% (berechnet aus den GC-peaks) und die spezifische Aktivität bezüglich der Cyclisierung von Homofarnesol wird mit 0,02mU/mg Protein angegeben.

Die Patentanmeldung JP2009060799 beschreibt die biokatalytische Synthese von (-)-Ambroxan aus Homofarnesol mittels einer SHC, wobei die Reaktion in einem Lösungsmittel bei einem pH von 5.2-6.6 stattfindet. Die experimentellen Beispiele verwenden die SHC aus A. acdidocaldarius.

Aufgabe der vorliegenden Erfindung war es somit ein neues Verfahren zur Herstellung von Ambroxan sowie Derivaten davon zur Verfügung zu stellen, das Vorteile gegenüber den technisch aufwendigen Synthesen des Standes der Technik bietet. Durch den Einsatz biotechnischer Verfahren sollte das Entstehen von Umweltgiften vermieden und der Energieaufwand drastisch reduziert werden. Weitere Aufgabe war es die anfallenden Kosten zusätzlich durch die Verwendung leicht zugänglicher Ausgangsstoffe sowie einer Verminderung der Anzahl chemischer Reaktionen (bzw. Stufen) zu senken. Ferner sollte eine erhöhte Produktivität erreicht werden.

Diese Aufgabe wird gelöst durch ein Verfahren zur Herstellung von Ambroxan-Derivaten, bevorzugt Ambroxan, der allgemeinen Formel (2) dadurch gekennzeichnet, dass Homofarnesol-Derivate der allgemeinen Formel (1) biokatalytisch zu den entsprechenden Ambroxan-Derivaten mittels eines Polypeptids mit der Aktivität einer Homofarnesol-Ambroxan-Cyclase als Enzym umgesetzt werden, wobei die Aktivität des Polypeptids die Umsetzung von Homofarnesol zu Ambroxan mit Hauptsubstrat Homofarnesol ist, dadurch gekennzeichnet, dass das Enzym ein Polypeptid ist, welches kodiert von einem Nukleinsäuremolekül wird, umfassend mindestens ein Nukleinsäuremolekül ausgewählt aus der Gruppe bestehend aus:
a) Nukleinsäuremolekül, das ein Polypeptid kodiert, umfassend SEQ ID NO 2, 5 oder 10;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 60% zu den Sequenzen SEQ ID NO 2, 5 oder 10 aufweist.

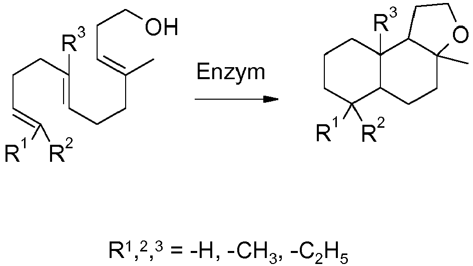

Derivate sind insbesondere Stereoisomere, bevorzugt Enantiomere aber auch Diastereomere der Verbindung (2). In einer Ausführung der vorliegenden Erfindung sind Derivate von Homofarnesol bzw. Ambroxan substituierte Verbindungen (1) und (2), wobei die Substituenten bezüglich der biokatalysierten Umsetzung inert sind. Damit sind insbesondere Verbindungen der unten dargestellten Strukturformeln gemeint.

Im Sinne der Erfindung werden die Begriffe "Verbindung (1), "Homofarnesol", "4,8,12-Trimethyltrideca-3,7,11-trien-1-ol)" und Derivate des Homofarnesols bzw. die Begriffe "Verbindung (2)", "Ambroxan", "Dodecahydronaphtho[2,1-b]furan" und Derivate des Ambroxans gleichbedeutend und gegeneinander austauschbar und ersetzbar, sofern nichts Gegenteiliges ausdrücklich definiert wird.

In einer bevorzugten Variante der Erfindung wird das linksdrehende Ambroxan der Formel ((-)-2) gebildet:

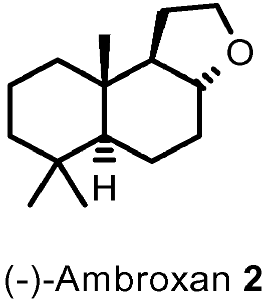

Polypeptide mit der Aktivität einer Homofarnesol-Ambroxan-Cyclase stellen eine neue Klasse von Enzymen dar.

Der Begriff "Aktivität" beschreibt die Fähigkeit eines Enzyms, ein Substrat in ein Produkt umzuwandeln. Die Aktivität kann in einem sogenannten Aktivitätstest über die Zunahme des Produktes, die Abnahme des Substrates (oder Eduktes) oder über eine Kombination dieser Parameter in Abhängigkeit von der Zeit bestimmt werden.

Die erfindungsgemäß verwendeten Enzyme besitzen eine Aktivität zur Umsetzung von Homofarnesol zu Ambroxan.

Im Sinne der Erfindung ist das Hauptsubstrat die chemische Verbindung, welche im Vergleich zu allen anderen Verbindungen, die von dem Enzym umgesetzt werden können, in Molprozent den größten Anteil als Reaktionspartner der Homofarnesol-Ambroxan-Cyclase aufweist.

Im Sinne der Erfindung ist das Hauptsubstrat Homofarnesol und somit die Hauptaktivität und damit die Hauptreaktion einer Homofarnesol-Ambroxan-Cyclase die Reaktion mit dem Hauptsubstrat Homofarnesol.

Die Aktivität der Homofarnesol-Ambroxan-Cyclase wird in einer Variante der Erfindung über die Ausbeute in Molprozent definiert. Bevorzugt erhält man bei der Umsetzung von Homofarnesol zu Ambroxan in Gegenwart einer Enzyms der neuen Klasse der Homofarnesol-Ambroxan-Cyclasen eine Ausbeute an Ambroxan in Molprozent bezogen auf die eingesetzten Mol Homofarnesol von 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100; besonders bevorzugt liegt die Ausbeute zwischen 5 und 100, 10 und 100, 20 und 100, 25 und 100, 30 und 100, 35 und 100, insbesondere zwischen 40 und 100, 45 und 100, 50 und 100, 60 und 100, 70 und 100 Molprozent.

Die Aktivität der Homofarnesol-Ambroxan-Cyclase wird in einer weiteren Variante der Erfindung über den Umsatz (Produktmenge/(Produktmenge + Resteduktmenge) * 100) in Molprozent definiert. Bevorzugt erhält man bei der Umsetzung von Homofarnesol zu Ambroxan in Gegenwart einer Enzyms der neuen Klasse der Homofarnesol-Ambroxan-Cyclasen einen Umsatz an Ambroxan in Molprozent von 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100; besonders bevorzugt liegt der Umsatz zwischen 5 und 100, 10 und 100, 20 und 100, 25 und 100, 30 und 100, 35 und 100, insbesondere zwischen 40 und 100, 45 und 100, 50 und 100, 60 und 100, 70 und 100 .

In einer bevorzugten Ausführung der Erfindung wird die Ausbeute und/oder der Umsatz über einen definierten Zeitraum von zum Beispiel 4, 6, 8, 10, 12, 16, 20, 24, 36 oder 48 Stunden bestimmt in dem die Umsetzung von Homofarnesol zu Ambroxan mittels der erfindungsgemäß verwendeten Cyclasen erfolgt. In einer weiteren Variante wird die Umsetzung unter genau definierten Bedingungen durchgeführt, von zum Beispiel 25, 30, 40, 50 oder 60°C. Insbesondere erfolgt die Bestimmung der Ausbeute und/oder des Umsatzes indem die Reaktion zur Umsetzung von Homofarnesol zu Ambroxan mittels der erfindungsgemäß verwendeten Cyclasen bei 30°C über 16 Stunden durchgeführt wird.
In einer Ausführung der Erfindung werden zur Bestimmung der Ausbeute und/oder des Umsatzes eine 10 mM Homofarnesol-Lösung (Citrat-gepuffert) mit einer Cyclase-Lösung umgesetzt wird, wobei das Enzym als Membran-Proteinextrakt Homofarnesol-Ambroxan-Cyclasen exprimierender Zellen (isoliert gemäß [Ochs D.et al.; J Bacteriol (1992), 174: 298]) in einer Konzentration an Proteingehalt von 0,08 Gewichtsprozent vorliegt.
In einer weiteren Ausführung der vorliegenden Erfindung wird eine Homofarnesol-Ambroxan-Cyclase dadurch gekennzeichnet, daß sie bei der Umsetzung von Homofarnesol zu Ambroxan unter den gleichen Bedingungen die 2-, 3-, 4-, 5-, 6-, 7-, 8-, 9-, 10-, 11-, 12-, 13-, 14-, 15-, 16-, 17-, 18-, 19-, 20-, 21-, 22-, 23-, 24-, 25-, 26-, 27-, 28-, 29-, 30-, 31-, 32-, 33-, 34-, 35-, 36-, 37-, 38-, 39-, 40-, 41-, 42-, 43-, 44-, 45-, 46-, 47-, 48-, 49-, 50-, 51-, 52-, 53-, 54-, 55-, 56-, 57-, 58-, 59-, 60-, 61-, 62-, 63-, 64-, 65-, 66-, 67-, 68-, 69-, 70-, 71-, 72-, 73-, 74-, 75-, 76-, 77-, 78-, 79-, 80-, 81-, 82-, 83-, 84-, 85-, 86-, 87-, 88-, 89-, 90-, 91-, 92-, 93-, 94-, 95-, 96-, 97-, 98-, 99-, 100-, 200-, 500-, 1000-fache oder höhere Ausbeute und/oder des Umsatz im Vergleich zu der Squalen-Hopen-Cyclase (SHC) aus *Alicyclobacillus acidocaldarius* (ehem. *Bacillus acidocaldarius*) zeigt. Der Begriff Bedingung bezieht sich hier auf Reaktionsbedingungen wie Susbstratkonzentration, Enzamkonzentration, Umsetzungszeitraum und/oder Temperatur.
In einer Variante der vorliegenden Erfindung wird eine Homofarnesol-Ambroxan-Cyclase durch jede oder mehrere, in jeder beliebigen Kombination, der oben genannten Definitionen charakterisiert.

Ebenfalls offenbart ist ein Verfahren zur Herstellung von Ambroxan, bei dem
a) Homofarnesol mit der Homofarnesol-Ambroxan-Cyclase in Kontakt gebracht und/oder inkubiert wird, und
b) Ambroxan isoliert wird.

In einer Ausführung der Erfindung wird Homofarnesol mit der Homofarnesol-Ambroxan-Cyclase in einem Medium in Kontakt gebracht und/oder so inkubiert, dass eine Umsetzung von Homofarnesol zu Ambroxan in Gegenwart der Cyclase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von vorzugsweise von 5 bis 8, aufweisen. Als Puffer kann ein Citrat-, Phosphat-, TRIS (Tris(hydroxymethyl)-aminomethan), MES (2-(N-Morpholino)ethansulfonsäure), Puffer verwendet werden. Ferner kann das Reaktionsmedium noch weitere Zusätze enthalten, wie z.B. Detergentien (beispielsweise Taurodeoxycholat).

Das Substrat (1) wird vorzugsweise in einer Konzentration von 5 - 100mM, besonders bevorzugt von 15 - 25mM in die enzymatische Umsetzung eingesetzt und kann kontinuierlich oder diskontinuierlich nachgeführt werden.

Die enzymatische Cyclisierung erfolgt in der Regel bei einer Reaktionstemperatur unterhalb der Desaktivierungstemperatur der eingesetzten Cyclase und oberhalb von -10 °C. Besonders bevorzugt liegt sie im Bereich von 0 bis 100 °C, insbesondere von 15 bis 60 °C und speziell von 20 bis 40 °C, z.B. bei etwa 30 °C.

Das Reaktionsprodukt Ambroxan kann mit organischen Lösungsmitteln, ausgewählt aus der Gruppe der unten genannten, extrahiert werden und gegebenenfalls zur Aufreinigung destilliert werden.

Neben diesen einphasigen wässrigen Systemen werden in einer weiteren Variante der Erfindung auch zweiphasige Systeme eingesetzt. Dabei werden ionische Flüssigkeiten als zweite Phase, bevorzugt aber organische, nicht-wasser-mischbare Reaktionsmedien als zweite Phase verwendet. Dadurch akkumulieren die Reaktionsprodukte in der organischen Phase. Nach der Reaktion ist Ambroxan in der organische Phase leicht von der wässrigen Phase, die den Biokatalysator enthält, abtrennbar.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

In einer Ausführung der vorliegenden Erfindung wird als Ausgangsstoff für die Ambroxan-Synthese Citral eingesetzt. Dies ist ein besonderer Vorteil des erfindungsgemäßen Verfahrens, da Citral in großen Mengen kostengünstig verfügbar ist. Citral wird in einer klassischen chemischen Synthese zu Homofarnesol umgesetzt

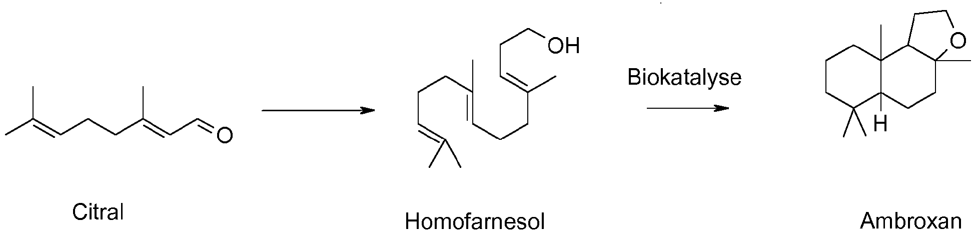

In einer Ausführung der vorliegenden Erfindung erfolgt die Umsetzung Citral zu Homofarnesol umgesetzt in folgenden Schritten (z.B.: JOC, (1992), 57, 2794):
Das erfindungsgemäße Verfahren bietet dadurch einen weiteren Vorteil, dass die gesamte Umsetzung von Homofarnesol zu Ambroxan in einphasigen wässrigen Systemen aber auch in 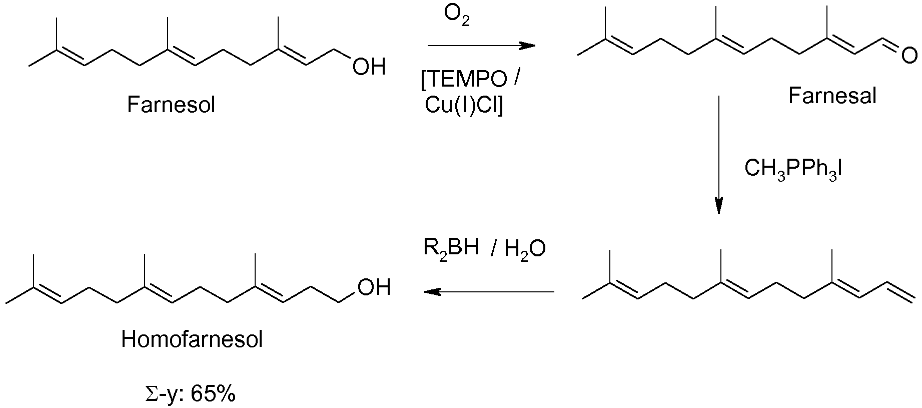 zweiphasigen Systemen stattfindet. Bei den zweiphasigen Systemen werden die oben genannten eingesetzt. Bevorzugt werden die oben genannten organische, nicht-wasser-mischbare Lösungsmittel als zweite Phase verwendet. Dadurch akkumulieren die Reaktionsprodukte in der organischen Phase. Nach der Reaktion ist Ambroxan in der organische Phase leicht von der wässrigen Phase, die den Biokatalysator enthält, abtrennbar.

Auch in der Ausführung der Erfindung mit Citral als Ausgangsstoff, wird Homofarnesol mit der Homofarnesol-Ambroxan-Cyclase in einem Medium in Kontakt gebracht und/oder so inkubiert, dass eine Umsetzung von Homofarnesol zu Ambroxan in Gegenwart der Cyclase erfolgt. Bevorzugt handelt es sich bei dem Medium um ein wässriges Reaktionsmedium. Bei den wässrigen Reaktionsmedien handelt es sich vorzugsweise um gepufferte Lösungen, die in der Regel einen pH-Wert von zugsweise von 5 bis 8, aufweisen. Als Puffer kann ein Citrat-, Phosphat-, TRIS (Tris(hydroxymethyl)-aminomethan), MES (2-(N-Morpholino)ethansulfonsäure) Puffer verwendet werden. Ferner kann das Reaktionsmedium noch weitere Zusätze enthalten, wie z.B. Detergentien (Taurodeoxycholat o.ä.).

In einer Ausführung wird das Reaktionsprodukt Ambroxan mit organischen Lösungsmitteln, ausgewählt aus der Gruppe der unten genannten, extrahiert werden und gegebenenfalls zur Aufreinigung destilliert werden.

Ebenfalls offenbart ist ein Verfahren zur biokatalytischen Herstellung von Ambroxan, dadurch gekennzeichnet, dass das Enzym ein Polypeptid ist, welches kodiert wird von einem Nukleinsäuremolekül umfassend mindestens ein Nukleinsäuremolekül ausgewählt aus der Gruppe bestehend aus:
a) Nukleinsäuremolekül, das ein Polypeptid kodiert, umfassend SEQ ID NO 2, 5, 6, 7, 8, 9, 10 oder 11;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
d) Nukleinsäuremolekül nach (a) bis (c), das für ein funktional äquivalentes Polypeptid oder ein Fragment der Sequenz gemäß SEQ ID NO 2, 5, 6, 7, 8, 9, 10 oder 11;
e) Nukleinsäuremolekül, kodierend für ein funktional äquivalentes Polypeptid mit der Aktivität einer Homofarnesol-Ambroxan-Cyclase, das man erhält in dem man ein Nukleinsäuremolekül aus einer cDNA Bank oder aus genomischer DNA mittels der Primer gemäß Sequenz Nr. 3 und 4 amplifiziert, oder das Nukleinsäuremolekül durch *de novo* Synthese chemisch synthetisiert;
f) Nukleinsäuremolekül, kodierend für ein funktional äquivalentes Polypeptid mit der Aktivität einer Homofarnesol-Ambroxan-Cyclase, das unter stringenten Bedingungen mit einem Nukleinsäuremolekül gemäß (a) bis (c) hybridisiert;
g) Nukleinsäuremolekül kodierend für ein funktional äquivalentes Polypeptid mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein funktional äquivalentes Polypeptid mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

Eine analoge oder ähnliche Bindungsstelle im obigen Sinne ist eine konservierte Domäne oder Motiv der Aminosäurensequenz mit einer Homologie von 80%, besonders bevorzugt 85%, 86%, 87%, 88%, 89%, 90%, insbesondere 91%, 92%, 93%, 94%, 95%, 96%, 97%, 98% oder 99% oder 100%, welche die Bindung desselben Substrats, insbesondere Homofarnesol gewährleistet.

Bevorzugt weist das Nukleinsäuremolekül c) eine Identität mit der SEQ ID NO:1 von mindestens 46%, 47%, 48%, 49%, 50%, 55%, 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% auf.

Ebenso weisen ein funktional äquivalentes Polypeptid eine Identität mit der SEQ ID NO: 2 von mindestens 46%, 47%, 48%, 49%, 50%, 55% 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% auf.

Anstelle des Begriffs "Identität" kann auch der Begriff "homolog" oder "Homologie" gleichbedeutend verwendet werden.

Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet.

Bevorzugt wird die Identität zwischen zwei Nukleinsäuresequenzen oder Polypeptidsequenzen durch die Identität der Nukleinsäuresequenz/Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453)) berechnet wird.
Bevorzugt werden die Identitätsvergleiche unter Einstellung folgender Parameter für Aminosäuren:
Gap creation penalty: 8; Gap extension penalty: 2
und die folgenden Parameter für Nukleinsäuren:

Gap creation penalty: 50; Gap extension penalty: 3 durchgeführt.
In einer Ausführung wird die Identität zwischen zwei Nukleinsäuresequenzen oder Polypeptidsequenzen durch Vergleich mit Hilfe des Programms BLASTP 2.2.20+ mit Standard-Einstellungen wie unter NCBI Blast vorgeschlagen (Reference: Altschul et al., (1997), Nucleic Acids Res. 25:3389-3402. Reference for compositional score matrix adjustment: Altschul et al., (2005), FEBS J. 272:5101-5109.)

Ebenfalls offenbart sind weitere Homologe oder funktionelle Äquivalente der SEQ ID NO: 1, die mit dieser Nukleinsäuresequenz unter stringenten Bedingungen hybridisieren.

"Funktionelle Äquivalente" beschreiben prinzipiell hier Nukleinsäuresequenzen, die unter Standardbedingungen mit einer Nukleinsäuresequenz oder Teilen einer Nukleinsäuresequenz hybridisieren und befähigt sind, die Expression eines Proteins mit den gleichen Eigenschaften wie die der Homofarnesol-Ambroxan-Cyclase in einer Zelle oder einem Organismus zu bewirken.

Zur Hybridisierung werden vorteilhaft kurze Oligonukleotide mit einer Länge von etwa 10-50 bp, vorzugsweise 15-40 bp beispielsweise der konservierten oder sonstigen Bereiche, die über Vergleiche mit anderen verwandten Genen in dem Fachmann bekannter Weise ermittelt werden können, verwendet. Es können aber auch längere Fragmente der erfindungsgemäßen Nukleinsäuren mit einer Länge von 100-500 bp oder die vollständigen Sequenzen für die Hybridisierung verwendet werden. Je nach der verwendeten Nukleinsäure/Oligonukleotid, der Länge des Fragmentes oder der vollständigen Sequenz oder je nachdem welche Nukleinsäureart, d.h. DNA oder RNA, für die Hybridisierung verwendet werden, variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardhybridisierungsbbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20°C bis 65°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 65°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50% in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, "Current Protocols in Molecular Biology", John Wiley & Sons, New York; Harnes and Higgins (eds), 1985, "Nucleic Acids Hybridization: A Practical Approach", IRL Press at Oxford University Press, Oxford; Brown (ed), 1991, Essential Molecular Biology: A Practical Approach, IRL Press at Oxford University Press, Oxford.

Unter einem funktionellen Äquivalent versteht man weiterhin auch Nukleinsäuresequenzen, die mit einer bestimmten Nukleinsäuresequenz ("ursprüngliche Nukleinsäuresequenz) bis zu einem definierten Prozentsatz homolog bzw. identisch sind und die gleiche Aktivität wie die ursprünglichen Nukleinsäuresequenzen aufweisen, ferner insbesondere auch natürliche oder künstliche Mutationen dieser Nukleinsäuresequenzen.

"Funktionale Äquivalente" oder Analoga der konkret offenbarten Enzyme sind im Rahmen der vorliegenden Offenbarung davon verschiedene Polypeptide, welche weiterhin die gewünschte biologische Aktivität, wie z.B. Hauptaktivität, Substratspezifität, besitzen. So versteht man beispielsweise unter "funktionalen Äquivalenten" Enzyme, die die Modellreaktion katalysieren und die mindestens 20 %, 30%, 50%, 60%, 65%, 70%, 75%, 80%, besonders bevorzugt 85%, 86%, 87%, 88%, 89%, 90%, insbesondere 91%, 92%, 93%, 94%, 95%, 96%, 97%, 98% oder 99% der Aktivität eines Enzyms, umfassend eine unter SEQ ID NO: 2, 5, 6, 7, 8, 9, 10 oder 11 aufgeführten Aminosäuresequenzen, aufweist.

Unter "funktionalen Äquivalenten" versteht man insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem hier offenbarten Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate".

"Präkursoren" sind dabei natürliche oder synthetische Vorstufen der Polypeptide mit oder ohne der gewünschten biologischen Aktiviät.

"Funktionale Derivate" der Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.
"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.
"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe des hier offenbarten Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Offenbart sind weiterhin Nukleinsäuresequenzen (einzel- und doppelsträngige DNA- und RNA-Sequenzen, wie z.B. cDNA und mRNA), die für ein Enzym mit erfindungsgemäßer Cyclase - Aktivität kodieren. Bevorzugt sind Nukleinsäuresequenzen, welche z.B. für Aminosäuresequenzen gemäß SEQ ID NO: 2, 5, 6, 7, 8, 9, 10 oder 11 charakteristische Teilsequenzen davon kodieren.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Weitere Ausgestaltungen zur Durchführung des erfindungsgemäßen biokatalytischen Verfahrens zur Herstellung von Ambroxan:
Das erfindungsgemäße Verfahren ist dadurch gekennzeichnet, dass das Enzym in einer Form vorliegt, ausgewählt aus der Gruppe bestehend aus:
a) freies, gegebenenfalls gereinigtes oder teilweise gereinigtes Polypeptid mit der Aktivität einer Homofarnesol-Ambroxan-Cyclase;
b) immobilisiertes Polypeptid mit der Aktivität einer Homofarnesol-Ambroxan-Cyclase;
c) aus Zellen isoliertes Polypeptid gemäß a) oder b);
d) ganze Zelle, gegebenenfalls ruhende oder aufgeschlossene Zellen, enthaltend mindestens ein Polypeptid mit der Aktivität einer Homofarnesol-Ambroxan-Cyclase;
e) Zelllysat oder Zellhomogenisat der Zellen beschrieben unter d).

In einer Ausführung der Erfindung sind die Zellen Mikroorganismen, bevorzugt transgene Mikroorganismen exprimierend mindestens ein heterologes Nukleinsäuremolekül kodierend für ein Polypeptid mit der Aktivität einer Homofarnesol-Ambroxan-Cyclase.

Weiterhin offenbart ist somit auch ein Genkonstrukt oder ein Vektor umfassend ein Nukleinsäuremolekül kodierend für ein Polypeptid mit der Aktivität einer Homofarnesol-Ambroxan-Cyclase, bevorzugt ein Nukleinsäuremolekül umfassend mindestens ein Nukleinsäuremolekül ausgewählt aus der Gruppe bestehend aus:
a) Nukleinsäuremolekül, das ein Polypeptid kodiert, umfassend SEQ ID NO 2, 5, 6, 7, 8, 9, 10 oder 11;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 46% zu den Sequenzen SEQ ID NO 2, 5, 6, 7, 8, 9, 10 oder 11 aufweist;
d) Nukleinsäuremolekül nach (a) bis (c), das für ein Fragment der Sequenzen gemäß SEQ ID NO 2, 5, 6, 7, 8, 9, 10 oder 11;
e) Nukleinsäuremolekül, kodierend für ein Polypeptids mit der Aktivität einer Homofarnesol-Ambroxan-Cyclase, das man erhält in dem man einen Nukleinsäuremolekül aus einer cDNA Datenbank oder aus einer Genom-Datenbank mittels der Primer gemäß Sequenz Nr. 3 und 4 amplifiziert;
f) Nukleinsäuremolekül, kodierend für ein Polypeptids mit der Aktivität einer Homofarnesol-Ambroxan-Cyclase, das unter stringenten Bedingungen mit einem Nukleinsäuremolekül gemäß (a) bis (c) hybridisiert;
g) Nukleinsäuremolekül kodierend für ein Polypeptids mit der Aktivität einer Homofarnesol-Ambroxan-Cyclase, das aus einer DNA-Bank unter Verwendung eines Nukleinsäuremoleküls gemäß (a) bis (c) oder deren Teilfragmente von mindestens 15 nt, vorzugsweise 20 nt, 30 nt, 50 nt, 100 nt, 200 nt oder 500 nt als Sonde unter stringenten Hybridisierungsbedingungen isoliert werden kann; und
h) Nukleinsäuremolekül, kodierend für ein Polypeptids mit der Aktivität einer Homofarnesol-Ambroxan-Cyclase, wobei die Sequenz des Polypeptids eine Identität von mindestens 46% zu den Sequenzen SEQ ID NO 2, 5, 6, 7, 8, 9, 10 oder 11 aufweist;
i) Nukleinsäuremolekül, kodierend für ein funktional äquivalentes Polypeptid mit der Aktivität einer Homofarnesol-Ambroxan-Cyclase, wobei das Polypeptid durch ein Nukleinsäuremolekül ausgewählt aus der Gruppe der in a) bis h) beschriebenen kodiert wird und mittels eines monoklonalen Antikörpers isoliert wurde oder isolierbar ist,
j) Nukleinsäuremolekül, kodierend für ein funktional äquivalentes Polypeptid mit der Aktivität einer Homofarnesol-Ambroxan-Cyclase, wobei das Polypeptid eine analoge oder ähnliche Bindungsstelle aufweist, wie ein Polypeptid kodiert durch ein Nukleinsäuremolekül ausgewählt aus der Gruppe der in a) bis h) beschriebenen.

Ferner sind die Wirtszellen enthaltend ein Genkonstrukt oder einen Vektor wie oben beschrieben ebenfalls hier offenbart. Hierzu werden die verwendeten Nukleinsäuresequenzen vorteilhaft in ein transgenes Genkostrukt eingebracht, die eine transgene Expression einer Homofarnesol-Ambroxan-Cyclase, in einem Organismus, bevorzugt Mikroorganismus gewährleisten können.

In den Genkostrukten steht dabei ein Nukleinsäuremolekül kodierend für eine Homofarnesol-Ambroxan-Cyclasebevorzugt in funktioneller Verknüpfung mit mindestens einem genetischen Kontrollelement (beispielsweise einem Promotor und/oder Terminator), das eine Expression in einem Organismus, bevorzugt Mikroorganismus gewährleistet.

Unter einer funktionellen Verknüpfung versteht man zum Beispiel die sequentielle Anordnung eines Promotors mit der zu exprimierenden Nukleinsäuresequenz kodierend für eine Homofarnesol-Ambroxan-Cyclase (zum Beispiel der Sequenz gemäß SEQ ID NO: 1) und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Die Herstellung einer funktionellen Verknüpfung als auch die Herstellung des Genkonstruktes kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann das Genkonstrukt, bestehend aus einer Verknüpfung von Promoter und zu exprimierender Nukleinsäuresequenz, integriert in einem Vektor vorliegen und durch zum Beispiel Transformation in einen Mikroorganismus insertiert werden.

Die in den Genkonstrukten oder Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluss auf das Zustandekommen oder die Funktion des Genkonstruktes haben. Genetische Kontrollsequenzen modifizieren zum Beispiel die Transkription und Translation in prokaryotischen oder eukaryotischen Organismen. Vorzugsweise umfassen die Genkonstrukte eine Kontrollsequenz, sowie gegebenenfalls weitere übliche regulative Elemente, und zwar jeweils funktionell verknüpft mit der transgen zu exprimierenden Nukleinsäuresequenz.

Als Kontrollsequenzen sind solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel die kodierende Sequenz eines bestimmten endogenen Gens gegen die für eine dsRNA kodierende Sequenz gezielt ausgetauscht werden.

Ein Genkonstrukt und die von ihr abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten, Vektoren oder transgenen Organismen haben. Beispielhaft aber nicht einschränkend seien zu nennen:
a) Selektionsmarker, die eine Resistenz gegen Antibiotika oder Biozide wie zum Beispiel Kanamycin, G 418, Bleomycin, Hygromycin, etc. verleihen.
b) Reportergene, die für leicht quantifizierbare Proteine kodieren und über Eigenfarbe oder Enzymaktivität eine Bewertung der Transformationseffizienz oder des Expressionsortes oder - zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al.(1995) Plant Journal 8(5):777-784), die Chloramphenicoltransferase, eine Luziferase (Ow et al. (1986) Science 234:856-859), das Aequorin-Gen (Prasher et al. (1985) Biochem Biophys Res Commun 126(3):1259-1268), die beta-Galactosidase, ganz besonders bevorzugt ist die β-Glucuronidase (Jefferson et al. (1987) EMBO J 6:3901-3907).
c) Replikationsursprünge, die eine Vermehrung der erfindungsgemäßen Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid oder ein Antibiotikum verleiht. Der Selektionsmarker erlaubt die Selektion der transformierten Zellen von untransformierten (McCormick et al. (1986) Plant Cell Reports 5:81-84).

Die Einführung des Genkonstruktes in einen Organismus kann vorteilhaft unter Verwendung von Vektoren realisiert werden, in denen die Genkonstrukte enthalten sind. Ein weiterer Gegenstand der Erfindung betrifft daher besagte transgene Vektoren, die ein transgens Genkonstrukt für eine Homofarnesol-Ambroxan-Cyclase umfassen.

Vektoren können beispielhaft Plasmide, Cosmide, Phagen, Viren sein. Das Genkonstrukt kann in den Vektor (bevorzugt ein Plasmidvektor) über eine geeignete Restriktionsschnittstelle eingeführt werden. Der entstandene Vektor wird zunächst in E.coli eingeführt. Korrekt transformierte E.coli werden selektioniert, gezüchtet und der rekombinante Vektor mit dem Fachmann geläufigen Methoden gewonnen. Restriktionsanalyse und Sequenzierung können dazu dienen, den Klonierungsschritt zu prüfen. Bevorzugt sind solche Vektoren, die eine stabile Integration der Expressionskassette in das Wirtsgenom ermöglichen.

Die Herstellung eines entsprechenden transgenen Organismus erfolgt z.B. mittels Transformation oder Transfektion mittels der entsprechenden Proteine oder Nukleinsäuren. Die Herstellung eines transformierten Organismus (bzw. einer transformierten Zelle) erfordert, dass die entsprechende DNA (z.B. der Expressionsvektor), RNA oder Protein in die entsprechende Wirtszelle eingebracht wird. Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika (wie Kanamycin, G 418, Bleomycin, Hygromycin etc.) zu verleihen vermag (s.o.). Transformierte Zellen, die ein solches Markergen exprimieren, sind in der Lage, in der Gegenwart von Konzentrationen eines entsprechenden Antibiotikums zu überleben, die einen untransformierten Wildtyp abtöten. 89:525-533 verwendet.

"Transgen" oder "recombinant" meint bezüglich zum Beispiel einer Nukleinsäuresequenz, eines Genkonstruktes oder einem Vektor enthaltend besagte Nukleinsäuresequenz oder einem Organismus transformiert mit besagter Nukleinsäuresequenz, Genkonstrukt oder Vektor alle solche durch gentechnische Methoden zustandegekommene Konstruktionen, in denen sich entweder
a) die Nukleinsäuresequenz kodierend für eine Homofarnesol-Ambroxan-Cyclase , oder
b) eine mit besagter Nukleinsäuresequenz unter a) funktionell verknüpfte genetische Kontrollsequenz, zum Beispiel ein funktioneller Promotor, oder
c) (a) und (b)

sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten.

Als transgene Organismen bevorzugte Wirts- oder Ausgangsorganismen sind vor allem Mikroorganismen gemäß der oben genannten Definition. Eingeschlossen sind im Rahmen der Erfindung insbesondere Mikroorganismen, bevorzugt solche transgenen oder rekombinanten Zellen, die aus Bakterien, Cyanobakterien, Pilzen und Hefen ausgewählt sind. Bevorzugt ist die Zelle ausgewählt aus Bakterien der Gattungen Escherichia, Corynebacterium, Ralstonia, Clostridium, Pseudomonas, Bacillus, Zymomonas, Rhodobacter, Streptomyces, Burkholderia, Lactobacillus und Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor und Zymomonas mobilis.

Als Spenderorganismus, d.h. Organismus aus dem eine Homofarnesol-Ambroxan-Cyclase isoliert wird, werden Methylococcus capsalatus, Rhodopseudomonas palustris, Bradyrhizobium japonicum, Frankia spec., Streptomyces coelicolor, Rhodopseudomonas palustris, Rhodopseudomonas palent, Frankia alni, Bacillus anthracis, Burkholderia ambifaria, insbesondere Zymomonas mobilis und Bradyrhizobium japonicum.

Die transgenen Organismen, insbesondere Streptomyces coelicolor oder Zymomonas mobilis, die endogene Homofarnesol-Ambroxan-Cyclasen aufweisen, zeigen in einer Variante der Erfindung eine Überexpression der Homofarnesol-Ambroxan-Cyclasen. Überexpression bedeutet jede Form der Expression der Homofarnesol-Ambroxan-Cyclasen die zusätzlich zu der ursprünglichen Expression des Wildtyps zu finden ist.

Ebenfalls offenbart ist die Herstellung transgener E. coli, die das Gen kodierend für die Homofarnesol-Ambroxan-Cyclasen exprimieren, bevorzugt SEQ ID NO 1. Hierzu wird mit Primern, z.B. Zm-SHC_fw und Zm-SHC_rev (SEQ ID NO 3 bzw. 3) das Gen der Cyclase, bevorzugt aus *Zymomonas mobilis* (SEQ ID NO 1) amplifiziert. Die Primer werden equimolar gemischt. Die PCR mit genomischer DNA eines Spendeorganismus, bevorzugt aus *Z. mobilis* (LU8910 = ATCC31821) werden nach Herstellerangaben mit Pwo-Polymerase (Roche Applied Science) und dem folgenden Temperatur-Gradienten-Programm durchgeführt: 95°C für 3 min; 30 Zyklen mit 95°C für 30 sec, 50°C für 30 sec, und 72°C für 3 min; 72°C für 10 min.; 4°C bis zur Verwendung. Das PCR-Produkt wird durch Agarosegel-Elektrophorese (1,2%E-Gel, Invitrogen) und Säulen-Chromatographie (GFX-Kit, Amersham Pharmacia) isoliert und anschließend sequenziert (Sequenzierprimer: Zm-SHC_fw und Zm-SHC_rev). Das PCR-Produkt wurde mit den Restriktionsendonukleasen, bevorzugt *Nde*I und *Bam*HI, verdaut und in entsprechend geschnittenen Vektor, bevorzugt *p*DHE1650-Vekto [WO 200132890 A1] ligiert wird. Das so erhaltene Plasmid wird in E. coli, bevorzugt in den Stamm *E. coli* TG10 *pA*gro4 [Takeshita S.et al.; Gene (1987), 61: 63] *p*HSG575 transformiert.

Aus einer entsprechenden Vorkultur angeimpft, werden die *E. coli* kultiviert, bevorzugt in LBAmp/Spec/Cm (100µg/l Ampicillin; 100µg/l Spectinomycin; 20µg/l Chloramphenicol), 0,1mM IPTG, 0,5g/L Rhamnose in 16 h bei 37°C angezogen, und anschließend bei 5000*g/10min zentrifugiert und gegebenenfalls bei 4°C gelagert.

In einer weiteren Ausführung wird die Cyclase aus dem Spendeorganismus oder aus dem transgenen Wirtsorganismus isoliert und gegebenenfalls gereinigt.

Aus den Zellen wird ein Proteinextrakt hergestellt indem das Zellpellet in Aufschlusspuffer (0,2M Tris/HCl, 0,5M EDTA, pH 8.0), 375U Benzonase (z.B. Novagen, 25U/µL), 40µL PMSF (100mM, gelöst in *i*-PropOH), 5,3g/100 ml Saccharose und ca. 3,3mg/100 ml Lysozym suspendiert wird. Den Ansatz wird gemischt und 30min auf Eis inkubiert. Anschließend wird gegebenenfalls die Mischung bei -20°C eingefroren werden. Nach dem Auftauen des Ansatzes wird mit Aqua dest. aufgefüllt und nochmals für 30min auf Eis inkubiert. Anschließend werden die Zellen 3mal 3min mit Ultraschall aufgeschlossen.

Nach dem Aufschluss wurden die Zelltrümmer in 60min bei 4°C und 26900 **g* abzentrifugiert. Der Überstand wird verworfen und das Pellet in Solubilisationspuffer (50mM Tris/HCl, 10mM MgCl₂x6H₂O, 1% Triton X-100, pH 8.0) resuspendiert und ca. 5min homogenisiert, z.B. mit einem Potter. Anschließend wird die Suspension für 30min auf Eis gehalten. Der homogenisierte Extrakt wird erneut für 1h bei 4°C und 26900 **g* zentrifugiert und das Pellet verworfen. Der Extrakt kann für Enzymtests eingesetzt werden und kann mehrere Wochen ohne Aktivitätsverlust bei -20°C gelagert werden. Der Proteingehalt liegt im Bereich von 1 mg /mL

Die erfindungsgemäß eingesetzten Cyclasen können im erfindungsgemäßen Verfahren als freies oder immobilisiertes Enzym verwendet werden.

Die erfindungsgemäß verwendeten Cyclasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Cyclase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Es können weiterhin ganze Zelle, gegebenenfalls ruhende oder aufgeschlossene Zellen, Zelllysat oder Zellhomogenisat verwendet werden.

Für das erfindungsgemäße Verfahren können wachsende Zellen verwendet werden, die für die Cyclase kodierenden Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Reaktion kann sowohl in *batch-* als auch in *fed*-*batch*-Fahrweise durchgeführt werden.

Das Produkt wird anschließend durch Extraktion und/oder Destillation gereinigt.

Weiterer Gegenstand der vorliegenden Erfindung ist die Verwendung eines Polypeptids mit der Aktivität einer Homofarnesol-Ambroxan-Cyclase zur biokatalytischen Umsetzung von Homofarnesol zu Ambroxan, dadurch gekennzeichnet, dass das Polypeptids kodiert wird von einem Nukleinsäuremolekül umfassend mindestens ein Nukleinsäuremolekül ausgewählt aus der Gruppe bestehend aus:
a) Nukleinsäuremolekül, das ein Polypeptid kodiert, umfassend SEQ ID NO 2, 5 oder 10;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 60% zu den Sequenzen SEQ ID NO 2, 5 oder 10 aufweist;
Die nachfolgenden Beispiele sollen die Erfindung veranschaulichen, ohne sie jedoch einzuschränken. Hierbei wird auf beiliegende Abbildungen Bezug genommen, dabei zeigt:

### Experimenteller Teil

### Beispiele

### Beispiel 1 Klonierung der Zm-SHC und Expression in E. coli

Mit den Oligonukleotiden Zm-SHC_fw und Zm-SHC_rev kann das Gen der Cyclase aus Zy*momonas mobilis* amplifiziert werden.

### Primer:

| Primer Nr. | Sequenz (5'->3') | Position |
|---|---|---|
| Zm-SHC_fw | gcgctgtttcatatgggtattgaca | N-Term-Primer |
| Zm-SHC_rev | gcgcttaccctggatcctcgaaaat | C-Term-Primer |

Je 100 ng der Primer Zm-SHC_fw und Zm-SHC_rev wurden equimolar gemischt. Die PCR mit genomischer DNA aus *Z. mobilis* (ATCC31821) wurde nach Herstellerangaben mit Pwo-Polymerase (Roche Applied Science) und dem folgenden Temperatur-Gradienten-Programm durchgeführt: 95°C für 3 min; 30 Zyklen mit 95°C für 30 sec, 50°C für 30 sec, und 72°C für 3 min; 72°C für 10 min.; 4°C bis zur Verwendung. Das PCR-Produkt (∼2.2 kB) wurde durch Agarosegel-Elektrophorese (1,2%E-Gel, Invitrogen) und Säulen-Chromatographie (GFX-Kit, Amersham Pharmacia) isoliert und anschließend sequenziert (Sequenzierprimer: Zm-SHC_fw und Zm-SHC_rev). Die erhaltene Sequenz entspricht der publizierten Sequenz.

Das PCR-Produkt wurde mit den Restriktionsendonukleasen *Nde*I und *Bam*HI verdaut und in entsprechend verdauten *p*DHE19.2-Vektor ligiert. Die Sequenzierung der resultierenden Plasmide ergab die in Seq-ID1 dargestellte Nukleinsäuresequenz.

Das Plasmid *p*DHE-Zm-SHC-1 wurde in den Stamm *E. coli* TG10 *p*Agro4 *p*HSG575 [Takeshita et al., Gene 1987, 61:63-74; Tomoyasu et al., Mol Microbiol 2001, 40:397-413] transformiert. Die rekombianten *E. coli* erhalten die Bezeichnung *E. coli* LU**15568.**

### Beispiel 2a: Bereitstellung rekombinanter Homofarnesol-Cyclase aus Z. mobilis (SEQ ID NO 2)

Aus einer entsprechenden 2mL Vorkultur angeimpft, wurde *E. coli* LU**15568** in 20mL LB-Amp/Spec/Cm (100µg/l Ampicillin; 100µg/l Spectinomycin; 20µg/l Chloramphenicol), 0,1 mM IPTG, 0,5g/L Rhamnose in 100mL Erlenmeyerkolben (Schikanen) 16 h bei 37°C angezogen, bei 5000*g/10min zentrifugiert und bei 4°C gelagert. Proteinextrakt wurde hergestellt indem das Zellpellet in 15mL Aufschlusspuffer (0,2M Tris/HCl, 0,5M EDTA, pH 8.0), 375U Benzonase (z.B. Novagen, 25U/µL), 40µL PMSF (100mM, gelöst in *i*-PropOH), 0,8g Saccharose und ca. 0.5mg Lysozym suspendiert wurden. Den Ansatz wurde gemischt und 30min auf Eis inkubiert. Anschließend wurde die Mischung bei -20°C eingefroren.

Nach dem Auftauen des Ansatzes wurden mit Aqua dest. auf ca. 40mL aufgefüllt und nochmals für 30 min auf Eis inkubiert.

Anschließend wurden die Zellen 3mal 3min mit Ultraschall aufgeschlossen (HTU-Soni 130, Fa. G. Heinemann, Schwäbisch-Hall, Amplitude 80%, 15" Puls / 15" Pause).

Nach dem Aufschluss wurden die Zelltrümmer in 60min bei 4°C und 26900 **g* abzentrifugiert. Der Überstand wurde verworfen und das Pellet in 100mL Solubilisationspuffer (50mM Tris/HCl, 10mM MgCl₂x6H₂O, 1% Triton X-100, pH 8.0) resuspendiert und ca. 5min gepottert. Anschließend wurde die Suspension für 30min auf Eis gehalten.

Der homogenisierte Extrakt wurde erneut für 1h bei 4°C und 26900 **g* zentrifugiert und das Pellet verworfen. Der Extrakt wurde für die Enzymtests eingesetzt und kann mehrere Wochen ohne Aktivitätsverlust bei -20°C gelagert werden. Der Proteingehalt liegt im Bereich von 1 mg /mL.

### Beispiel 2b: Bereitstellung rekombinanter Homofarnesol-Cyclase aus Bradyrhizobium japonicum (SEQ ID NO 5)

Homofarnesol-Cyclase aus Bradyrhizobium japonicum wurde wie in Beispiel 2a hergestellt.

### Beispiel 3a: Aktivitätsbestimmung der rekombinanten Homofarnesol-Cyclase (SEQ ID NO 2) aus E. coli LU15568

Mit der in Beispiel 2a beschriebenen Proteinpräparation wurde Homofarnesol (**1**, (3Z,7E)-4,8,12-Trimethyltrideca-3,7,11-trien-1-ol) inkubiert. Im Einzelnen wurden 4mL Proteinpräparation, 0.5mL Na-Citratpuffer (1M Natriumcitrat pH 4.9), 0.5mL Homofarnesol-Lösung (100mM in 0.1 M Na-Citratpuffer, pH 6.5 mit 2%(w/w) Taurodesoxycholat) miteinander gemischt und unter Rühren bei 30°C inkubiert. Eine Kontrollreaktion wurde in gleicher Zusammensetzung jedoch bei 60°C inkubiert.

Nach einer 16stündigen Inkubation wurden die Ansätze mit 10mL Hexan/ *n*-Propanol 3:2 extrahiert und die organische Phase zu Trockne eingeengt.

Der Rückstand wurde in 200µL Dichlormethan aufgenommen und für die GC bzw. GC/MS-Analyse eingesetzt.

Mit der in Beispiel 4 dargestellten Analytik konnte ein Umsatz von 41% (»20.5 µmol **2**) festgestellt werden.

Im Vergleich wurde in Umsetzungen von Homofarnesol mit der bekannten Squalen-Hopen-Cyclase aus Alicyclobacillus acidocaldarius 1.2% Umsatz erzielt.

### Beispiel 3b: Aktivitätsbestimmung der rekombinanten Homofarnesol-Cyclase (SEQ ID NO 5) aus E. coli

Die enzymatische Aktivität des in E. coli rekombinant hergestellten Biokatalysators aus Bradyrhizobium japonicum entspricht der Zm-SHC. Nach 3stündiger Inkubation einer 20mM Homofarnesol Lösung mit Zellhomogenat (Proteinmenge: 31 mg) bei 37 °C wurden 26,4% Homofarnesol zum Ambroxan umgesetzt. Unter den gleichen Bedingungen wurden mit rekombinanter Zm-SHC einen Umsatz von 22,4% erzielt.

### Beispiel 4 Analytik

### Umsatz/Quantifizierung

Der Umsatz von Homofarnesol (**1**) zu Ambroxan (**2**) lässt sich mit folgendem GC-System bestimmen:
*Säule:* 10m Optima 1

### Temeraturrofil:

| | | |
|---|---|---|
| 0': | 100°C | |
| | | 15°C/min |
| 14.7': | 320°C | |
| 34.7' | 320°C | |
| | | |
| *Injektortemp*: | | 320°C |

*RT*: Homofarnesol ca. 12.5'; Ambroxan: ca. 11.4'

Mit authentischem Material wurde eine Eichreihe erstellt, anhand derer die Konzentration unbekannter Proben bestimmt wurde.

### Identifizierung

Die Identifizierung erfolgte mittels Kapillar-GC/MS der positiven Ionen nach Elektronenstoßionisation (EI) und chemischer Ionisation (CI) mit Ammoniak. Geräte: GC (HP 6890) gekoppelt mit zwei MSDs (HP 5973) für EI and CI Ionisation.

### GC BEDINGUNGEN:

| | |
|---|---|
| Trennsäule: | DB-1 |
| Länge: | 30m |
| Innendurchmesser: | 250µm |
| Filmdicke | 0.25µm |
| Trägergas: | He |
| Fluss: | 1.2mL/min |
| Splitverhältnis: | 1:60 |
| Ofentemperatur: | 100°C, 5K/min bis 200°C, 5 min isotherm, 30K/min bis 300°C, 50 min isotherm |
| Injektortemperatur: | 250°C |
| Transferlinetemperatur: | 300°C |
| Dosiermenge: | 0.2µL |
| Probenvorbereitung: | Direkte Injektion |

### MS BEDINGUNGEN

| EI: | | CI: | |
|---|---|---|---|
| Scanbereich: | 25- 750 amu | Scanbereich: | 75-800 amu |
| Ionisierungsenergie: | 70 eV | | |

## Patentansprüche

1. Verfahren zur Herstellung von Ambroxan-Derivaten, bevorzugt Ambroxan, der allgemeinen Formel (2) **dadurch gekennzeichnet, dass** Homofamesol-Derivate der allgemeinen Formel (1) biokatalytisch zu den entsprechenden Ambroxan-Derivaten mittels eines Polypeptid mit der Aktivität einer Homofarnesol-Ambroxan-Cyclase als Enzym umgesetzt werden, 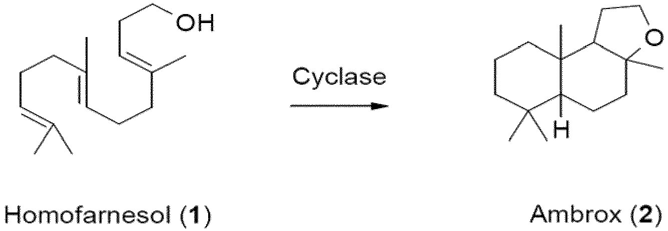 wobei die Aktivität des Polypeptids die Umsetzung von Homofarnesol zu Ambroxan mit Hauptsubstrat Homofarnesol ist, **dadurch gekennzeichnet, dass** das Enzym ein Polypeptid ist, welches kodiert von einem Nukleinsäuremolekül wird, umfassend mindestens ein Nukleinsäuremolekül ausgewählt aus der Gruppe bestehend aus:
a) Nukleinsäuremolekül, das ein Polypeptid kodiert, umfassend SEQ ID NO 2, 5 oder 10;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 60% zu den Sequenzen SEQ ID NO 2, 5 oder 10 aufweist.

2. Verfahren nach Anspruch 1, **dadurch gekennzeichnet, dass** die Homofarnesol-Ambroxan-Cyclasen einen Umsatz an Ambroxan in Molprozent von 10 bis 100 aufweisen.

3. Verfahren nach einem der vorhergehenden Ansprüche, **dadurch gekennzeichnet, dass** das Enzym in einer Form vorliegt, ausgewählt aus der Gruppe bestehend aus:
a) freies, gegebenenfalls gereinigtes oder teilweise gereinigtes Polypeptid mit der Aktivität einer Homofarnesol-Ambroxan-Cyclase;
b) immobilisiertes Polypeptid mit der Aktivität einer Homofarnesol-Ambroxan-Cyclase;
c) aus Zellen isoliertes Polypeptid gemäß a) oder b);
d) ganze Zelle, gegebenenfalls ruhende oder aufgeschlossene Zellen, enthaltend mindestens ein Polypeptid mit der Aktivität einer Homofarnesol-Ambroxan-Cyclase;
e) Zelllysat oder Zellhomogenisat der Zellen beschrieben unter d).

4. Verfahren nach Anspruch 1, **dadurch gekennzeichnet, dass** die Zellen Mikroorganismen sind, bevorzugt transgene Mikroorganismen exprimierend mindestens ein heterologes Nukleinsäuremolekül kodierend für ein Polypeptid mit der Aktivität einer Homofarnesol-Ambroxan-Cyclase.

5. Verfahren nach einem der vorhergehenden Ansprüche, **dadurch gekennzeichnet, dass** zur Herstellung von Ambroxan als Ausgangsstoff Citral eingesetzt wird, welches in mehreren Schritten zu Homofarnesol umgesetzt wird.

6. Verfahren nach einem der vorhergehenden Ansprüche, **dadurch gekennzeichnet, dass** die Herstellung von Ambroxan in einphasigen wässrigen Systemen oder in zweiphasigen Systemen erfolgt.

7. Verfahren nach einem der vorhergehenden Ansprüche, **dadurch gekennzeichnet, dass** die Herstellung von Ambroxan in batch-, fed-batch oder kontinuierlicher Fahrweise erfolgt.

8. Verfahren nach einem der vorhergehenden Ansprüche, wobei die Reaktion von Homofarnesol zu Ambroxan bei einer Temperatur im Bereich von 0 bis 60°C und/oder einem pH-Wert im Bereich von 4 bis 8 erfolgt.

9. Verfahren nach Anspruch 8, **dadurch gekennzeichnet, dass** die Reaktion von Homofarnesol zu Ambroxan bei einer Temperatur im Bereich von 0 bis 45°C erfolgt.

10. Verfahren nach einem der vorhergehenden Ansprüche, **dadurch gekennzeichnet, dass** die Homofarnesol-Ambroxan-Cyclase aus einem Mikroorganismus ausgewählt aus der Gruppe bestehend aus Zymomonas mobilis, Bradyrhizobium japonicum und Rhodopseudomonas palustris isoliert wurde.

11. Verfahren nach einem der vorhergehenden Ansprüche, **dadurch gekennzeichnet, dass** die Homofarnesol-Ambroxan-Cyclase aus transgenen Bakterien der Spezies Escherichia coli, Pseudomonas putida, Burkholderia glumae, Streptomyces lividans, Streptomyces coelicolor und Methylococcus capsalatus, Rhodopseudomonas palustris, Frankia spec., Rhodop-seudomonas palent, Frankia alni, Bacillus anthracis, Burkholderia ambifaria, insbesondere Zymomonas mobilis und Bradyrhizobium japonicum isoliert wurde, welche die Homofarnesol-Ambroxan-Cyclase überexprimieren.

12. Verwendung eines Polypeptids mit der Aktivität einer Homofarnesol-Ambroxan-Cyclase zur biokatalytischen Umsetzung von Homofarnesol zu Ambroxan, **dadurch gekennzeichnet, dass** das Polypeptids kodiert wird von einem Nukleinsäuremolekül umfassend mindestens ein Nukleinsäuremolekül ausgewählt aus der Gruppe bestehend aus:
a) Nukleinsäuremolekül, das ein Polypeptid kodiert, umfassend SEQ ID NO 2, 5 oder 10;
b) Nukleinsäuremolekül, das zumindest ein Polynukleotid der Sequenz gezeigt in SEQ ID NO 1 umfasst;
c) Nukleinsäuremolekül, das ein Polypeptid kodiert, dessen Sequenz eine Identität von mindestens 60% zu den Sequenzen SEQ ID NO 2, 5 oder 10 aufweist.

## Claims

1. A process for the production of ambroxan derivatives, preferably ambroxan, of the general formula (2), wherein homofarnesol derivatives of the general formula (1) are reacted biocatalytically by means of a polypeptide with the activity of a homofarnesol-ambroxan cyclase as the enzyme to give the corresponding ambroxan derivatives, 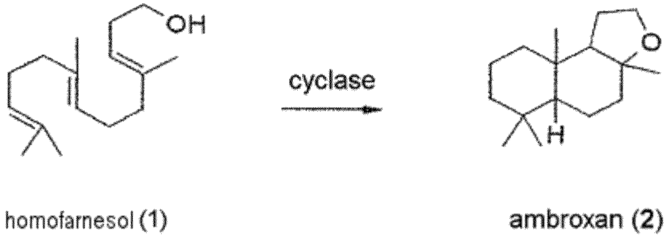 where the activity of the polypeptide is the reaction of homofarnesol to give ambroxan with main substrate homofarnesol, wherein the enzyme is a polypeptide which is encoded by a nucleic acid molecule comprising at least one nucleic acid molecule selected from the group consisting of:
a) nucleic acid molecule which codes for a polypeptide comprising the SEQ ID NO 2, 5 or 10;
b) nucleic acid molecule which comprises at least one polynucleotide of the sequence shown in SEQ ID NO 1;
c) nucleic acid molecule which codes for a polypeptide whose sequence has an identity of at least 60% to the sequences SEQ ID NO 2, 5 or 10.

2. The process according to claim 1, wherein the homofarnesol-ambroxan cyclases have an ambroxan reaction rate in mol percent of from 10 to 100.

3. The process according to either of the preceding claims, wherein the enzyme is present in a form selected from the group consisting of:
a) free, optionally purified or partially purified polypeptide with the activity of a homofarnesol-ambroxan cyclase;
b) immobilized polypeptide with the activity of a homofarnesol-ambroxan cyclase;
c) polypeptide, isolated from cells, according to a) or b);
d) intact cell, optionally quiescent or disrupted cells, comprising at least one polypeptide with the activity of a homofarnesol-ambroxan cyclase;
e) cell lysate or cell homogenate of the cells described under d).

4. The process according to claim 1, wherein the cells are microorganisms, preferably transgenic microorganisms, expressing at least one heterologous nucleic acid molecule coding for a polypeptide with the activity of a homofarnesol-ambroxan cyclase.

5. The process according to any of the preceding claims, wherein the starting material employed for the production of ambroxan is citral, which is reacted in a plurality of steps to give homofarnesol.

6. The process according to any of the preceding claims, wherein ambroxan is produced in single-phase aqueous systems or in two-phase systems.

7. The process according to any of the preceding claims, wherein ambroxan is produced batchwise, fed-batch-wise or continuously.

8. The process according to any of the preceding claims, wherein the reaction of homofarnesol to ambroxan takes place at a temperature in the range of from 0 to 60°C and/or at a pH in the range of from 4 to 8.

9. The process according to claim 8, wherein the reaction of homofarnesol to ambroxan takes place at a temperature in the range of from 0 to 45°C.

10. The process according to one of the preceding claims, wherein homofarnesol-ambroxan cyclase has been isolated from a microorganism selected from the group consisting of Zymomonas mobilis, Bradyrhizobium japonicum and Rhodopseudomonas palustris,

11. The process according to one of the preceding claims, wherein homofarnesol-ambroxan cyclase has been isolated from transgenic bacteria of the species Escherichia coli, Pseudomonas putida, Burkholderia glumae, Streptomyces lividans, Streptomyces coelicolor and Methylococcus capsalatus, Rhodopseudomonas palustris, Frankia spec., Rhodopseudomonas palent, Frankia alni, Bacillus anthracis, Burkholderia ambifaria, in particular Zymomonas mobilis and Bradyrhizobium japonicum, which overexpress homofarnesol-ambroxan cyclase.

12. Use of a polypeptide with the activity of a homofarnesolambroxan cyclase for the biocatalytic reaction of homofarnesol to give ambroxan, wherein the polypeptide is encoded by a nucleic acid molecule comprising at least one nucleic acid molecule selected from the group consisting of:
a) nucleic acid molecule which codes for a polypeptide comprising the SEQ ID NO 2, 5 or 10;
b) nucleic acid molecule which comprises at least one polynucleotide of the sequence shown in SEQ ID NO 1;
c) nucleic acid molecule which codes for a polypeptide whose sequence has an identity of at least 60% to the sequences SEQ ID NO 2, 5 or 10.

## Revendications

1. Procédé de fabrication de dérivés d'ambroxan, de préférence d'ambroxan, de formule générale (2), **caractérisé en ce que** des dérivés d'homofarnésol de formule générale (1) sont transformés biocatalytiquement en les dérivés d'ambroxan correspondants au moyen d'un polypeptide ayant l'activité d'une homofarnésol-ambroxan-cyclase en tant qu'enzyme, 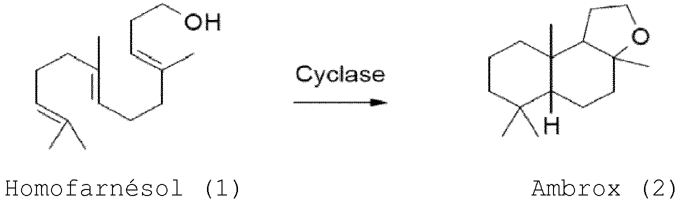 l'activité du polypeptide étant la transformation de l'homofarnésol en ambroxan avec comme substrat principal l'homofarnésol,
**caractérisé en ce que** l'enzyme est un polypeptide qui est codé par une molécule d'acide nucléique, comprenant au moins une molécule d'acide nucléique choisie dans le groupe constitué par :
a) une molécule d'acide nucléique qui code pour un polypeptide comprenant la SEQ ID NO 2, 5 ou 10 ;
b) une molécule d'acide nucléique qui comprend au moins un polynucléotide de la séquence indiquée dans SEQ ID NO 1 ;
c) une molécule d'acide nucléique qui code pour un polypeptide dont la séquence présente une identité d'au moins 60 % avec les séquences SEQ ID NO 2, 5 ou 10.

2. Procédé selon la revendication 1, **caractérisé en ce que** les homofarnésol-ambroxan-cyclases présentent une conversion en ambroxan en pourcentage en moles de 10 à 100.

3. Procédé selon l'une quelconque des revendications précédentes, **caractérisé en ce que** l'enzyme se présente sous une forme choisie dans le groupe constitué par :
a) un polypeptide libre, éventuellement purifié ou partiellement purifié, ayant l'activité d'une homofarnésol-ambroxan-cyclase ;
b) un polypeptide immobilisé ayant l'activité d'une homofarnésol-ambroxan-cyclase ;
c) un polypeptide selon a) ou b) isolé à partir de cellules ;
d) des cellules entières, éventuellement des cellules au repos ou décomposées, contenant au moins un polypeptide ayant l'activité d'une homofarnésol-ambroxan-cyclase ;
e) un lysat cellulaire ou un homogénat cellulaire des cellules décrites en d).

4. Procédé selon la revendication 1, **caractérisé en ce que** les cellules sont des microorganismes, de préférence des microorganismes transgéniques exprimant au moins une molécule d'acide nucléique hétérologue codant pour un polypeptide ayant l'activité d'une homofarnésol-ambroxan-cyclase.

5. Procédé selon l'une quelconque des revendications précédentes, **caractérisé en ce que** du citral, qui est transformé en homofarnésol en plusieurs étapes, est utilisé en tant que matière première pour la fabrication d'ambroxan.

6. Procédé selon l'une quelconque des revendications précédentes, **caractérisé en ce que** la fabrication d'ambroxan a lieu dans des systèmes aqueux monophasés ou dans des systèmes biphasés.

7. Procédé selon l'une quelconque des revendications précédentes, **caractérisé en ce que** la fabrication d'ambroxan a lieu en mode discontinu, en mode d'alimentation discontinue ou en mode continu.

8. Procédé selon l'une quelconque des revendications précédentes, **caractérisé en ce que** la réaction d'homofarnésol en ambroxan a lieu à une température dans la plage allant de 0 à 60 °C et/ou à un pH dans la plage allant de 4 à 8.

9. Procédé selon la revendication 8, **caractérisé en ce que** la réaction d'homofarnésol en ambroxan a lieu à une température dans la plage allant de 0 à 45 °C.

10. Procédé selon l'une quelconque des revendications précédentes, **caractérisé en ce que** l'homofarnésol-ambroxan-cyclase a été isolée à partir d'un microorganisme choisi dans le groupe constitué par Zymomonas mobilis, Bradyrhizobium japonicum et Rhodopseudomonas palustris.

11. Procédé selon l'une quelconque des revendications précédentes, **caractérisé en ce que** l'homofarnésol-ambroxan-cyclase a été isolée à partir de bactéries transgéniques des espèces Escherichia coli, Pseudomonas putida, Burkholderia glumae, Streptomyces lividans, Streptomyces coelicolor et Methylococcus capsalatus, Rhodopseudomonas palustris, Frankia spec., Rhodopseudomonas palent, Frankia alni, Bacillus anthracis, Burkholderia ambifaria, notamment Zymomonas mobilis et Bradyrhizobium japonicum, qui surexpriment l'homofarnésol-ambroxan-cyclase.

12. Utilisation d'un polypeptide ayant l'activité d'une homofarnésol-ambroxan-cyclase pour la transformation biocatalytique d'homofarnésol en ambroxan, **caractérisée en ce que** le polypeptide est codé par une molécule d'acide nucléique comprenant au moins une molécule d'acide nucléique choisie dans le groupe constitué par :
a) une molécule d'acide nucléique qui code pour un polypeptide comprenant la SEQ ID NO 2, 5 ou 10 ;
b) une molécule d'acide nucléique qui comprend au moins un polynucléotide de la séquence indiquée dans SEQ ID NO 1 ;
c) une molécule d'acide nucléique qui code pour un polypeptide dont la séquence présence une identité d'au moins 60 % avec les séquences SEQ ID NO 2, 5 ou 10.
